# EUROPEAN PATENT APPLICATION

(11) **EP 4 378 940 A1**
(43) Date of publication of application: **05.06.2024**
(21) Application number: 22832064.4
(22) Date of filing: 28.06.2022
(51) Int. Cl.: C07D 409/14, C07D 417/14, C07D 403/14, C07D 413/14, C07D 405/14, C07D 403/12, C07D 401/14, C07D 471/04, A61P 35/00, A61P 29/00, A61P 31/14, A61K 31/4184, A61K 31/427, A61K 31/437, A61K 31/506, A61K 31/422, A61K 31/4439

(54) **POLYCYCLIC COMPOUND FOR INHIBITING RNA HELICASE DHX33, AND APPLICATION OF COMPOUND**

(30) Priority: 29.06.2021 CN 202110724794
(71) Applicant: Shenzhen Keye Life Technologies, Co., Ltd., Shenzhen, Guangdong 518122 (CN)
(72) Inventor: ZHANG, Yandong, Shenzhen, Guangdong 518122 (CN); LI, Xianglu, Shenzhen, Guangdong 518122 (CN)
(74) Representative: Cabinet Laurent & Charras
(86) International application number: PCT/CN2022/102010
(87) International publication number: WO 2023/274251

(57) **Abstract**

The present invention relates to a polycyclic compound for inhibiting RNA helicase DHX33, and an application of the compound. In particular, the present invention relates to a compound as represented by formula I or a pharmaceutically acceptable form thereof, a pharmaceutical composition comprising the same, a preparation method therefor, and a medical use thereof for preventing and/or treating DHX33-associated diseases.

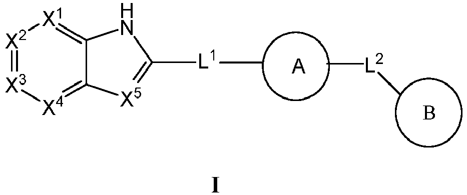

## Description

### TECHNICAL FIELD

The present disclosure belongs to the field of medicinal chemistry, and relates to a small molecule inhibitor of DHX33, a pharmaceutical composition comprising the same, a preparation method thereof and a medical use thereof for preventing and/or treating DHX33-related diseases.

### BACKGROUND

The present disclosure relates to compounds that inhibit the RNA helicase activity of DHX33. DHX33 belongs to the DEAD/H-box protein family of RNA helicases, wherein DEAD/H represents the abbreviation of the amino acid sequence, i.e., Asp-Glu-Ala-Asp/His. This sequence, together with many other conservative amino acid sequences, appears in the protein sequences of the members of the RNA helicase family and is highly involved in the binding with nucleic acid substrates and ATP hydrolysis. Although these family members have these same sequences in common, each RNA helicase has particular specificity and unique biological function. Human DHX33 protein has a molecular weight of approximately 72 kDa and has the function of unwinding nucleic acids. It utilizes the biological energy released by ATP hydrolysis to drive changes in the conformation of the complex of RNA and protein, thus participating in a variety of metabolic activities of RNA, specifically, a series of biological processes such as the transcription, splicing, editing, translation and degradation of RNA. The function of DHX33 is not merely limited to the modification of RNA molecules. Studies have demonstrated that, in addition to unwinding the two strands of RNA, DHX33 protein is also involved in the metabolism of DNA. Specifically, DHX33 protein is capable of unwinding the double-stranded structure of DNA and playing an important role in the process of gene expression.

Studies have demonstrated that, by binding to a variety of cancer-related gene promoters, DHX33 affects the methylation status of DNA and thus regulates the expression of a variety of cancer genes and the signaling pathways related to the development of tumor at genome level, which plays a crucial role in a variety of cellular activities such as the growth, proliferation, migration, apoptosis and glycometabolism of cells. In addition, it has been found that DHX33 is capable of sensing the invasion of foreign double-stranded RNA molecules and playing an important role in the innate immunity of cells. As a very important cell growth regulatory gene, DHX33 is highly expressed in a variety of cancers such as lung cancer, lymphoma, glioblastoma, breast cancer, colon cancer, liver cancer. The occurrence and development of a variety of cancers depend on the high expression of DHX33 protein. Genetic knockout of DHX33 is capable of significantly inhibiting the occurrence and development of the lung cancer driven by RAS oncogene. It has been confirmed by in-vivo and in-vitro experiments that, upon the inhibition of DHX33 protein, the occurrence and development of a variety of cancers such as breast cancer, colon cancer, brain glioma and lymphoma are inhibited significantly.

Studies have demonstrated that the function of DHX33 protein depends on its helicase activity. A DHX33 mutant lacking the helicase activity does not have the function of DHX33 protein, and the function of the wild-type DHX33 gene cannot be replaced. The present disclosure provides the structures and the synthetic methods of a variety of compounds capable of inhibiting the enzymatic activity of DHX33, and these compounds possess uses in the preparation of a drug for treating a disease or a disorder at least partially mediated by DHX33.

### SUMMARY

In the present disclosure, a series of small molecule compounds that inhibit the RNA helicase activity of DHX33 have been found through extensive studies, and these compounds have potential value in preventing and/or treating DHX33-related diseases.

In a first aspect, the present disclosure provides a compound having the structure of formula I or a pharmaceutically acceptable form thereof: 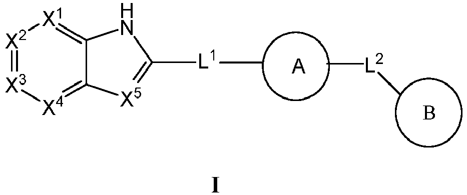 wherein
X¹ is N or CR¹, X² is N or CR², X³ is N or CR³, X⁴ is N or CR⁴;
R¹, R², R³ and R⁴ are each independently hydrogen, halogen, amino, nitro, hydroxyl, C₁₋₆ alkyl, C₁₋₆ haloalkyl, C₁₋₆ alkoxy, C₁₋₆ haloalkoxy, -NH(C₁₋₆ alkyl), -N(C₁₋₆ alkyl)₂, C₁₋₆ hydroxyalkyl, -O-(C₁₋₆ alkylene)-O-(C₁₋₆ alkyl), -C(=O)-NH-(C₁₋₆ alkyl), -C(=O)-NH-(C₁₋₆ alkylene)-N(C₁₋₆ alkyl)₂, or -C(=O)-O-(C₁₋₆ alkyl);
X⁵ is N or CR⁵, R⁵ is hydrogen, halogen, or C₁₋₆ alkyl, and the C₁₋₆ alkyl is optionally substituted with one or more substituent selected from halogen, C₁₋₆ alkyl, -(C₁₋₆ alkylene)-O-(C₁₋₆ alkyl), or -(C₁₋₆ alkylene)-O-C(=O)-(C₁₋₆ alkyl);
L¹ is -NR⁶C(=O)-, -NR⁶S(=O)₂-, -NR⁶S(=O)-, -C(=O)NR⁶-, -S(=O)₂NR⁶-, or - S(=O)NR⁶-;
each R⁶ is independently hydrogen, C₁₋₆ alkyl, C₃₋₈ cycloalkyl, or C₆₋₁₀ aryl;
ring A is a 5-10 membered heteroaryl or a 3-8 membered heterocyclyl, ring A is optionally substituted with one or more R⁷;
each R⁷ is independently halogen, C₁₋₆ alkyl, or C₁₋₆ haloalkyl;
L² is a single bond, O, S, or CR⁸R⁹;
R⁸ and R⁹ are each independently hydrogen, halogen, or C₁₋₆ alkyl;
ring B is C₆₋₁₀ aryl, a 5-12 membered heteroaryl, or a 3-8 membered heterocyclyl, ring B is optionally substituted with one or more R¹⁰;
each R¹⁰ is independently halogen, cyano, amino, nitro, hydroxyl, C₁₋₆ alkyl, C₁₋₆ haloalkyl, C₁₋₆ alkoxy, C₁₋₆ haloalkoxy, C₃₋₆ cycloalkyl, -C(=O)-O-(C₁₋₆ alkyl), phenyl, benzyl, pyridyl, -C(=O)-NH₂, or -NH-C(=O)-(C₁₋₆ alkyl), and the phenyl, benzyl, or pyridyl is optionally substituted with one or more substituents selected from hydrogen, halogen, cyano, amino, hydroxyl, C₁₋₆ alkyl, orC₁₋₆ alkoxy;
said pharmaceutically acceptable form is selected from a pharmaceutically acceptable salt, an ester, a stereoisomer, a tautomer, a solvate, a N-oxide, an isotopically labeled form, a metabolite and a prodrug.

In some embodiments, R¹, R², R³ and R⁴ in the above-mentioned compound of formula I or the pharmaceutically acceptable form thereof are each independently hydrogen, halogen, amino, nitro, hydroxyl, C₁₋₆ alkyl, C₁₋₆ haloalkyl, C₁₋₆ alkoxy, C₁₋₆ haloalkoxy, -NH(C₁₋₆ alkyl), -N(C₁₋₆ alkyl)₂, or C₁₋₆ hydroxyalkyl.

In some preferred embodiments, R¹, R², R³ and R⁴ in the above-mentioned compound of formula I or the pharmaceutically acceptable form thereof are each independently hydrogen, halogen, amino, nitro, hydroxyl, C₁₋₄ alkyl, C₁₋₄ haloalkyl, C₁₋₄ alkoxy, or C₁₋₄ haloalkoxy.

In some more preferred embodiments, R¹, R², R³ and R⁴ in the above-mentioned compound of formula I or the pharmaceutically acceptable form thereof are each independently hydrogen, halogen, amino, nitro, hydroxyl, methyl, methoxy, or trifluoromethoxy.

In some embodiments, R⁵ in the above-mentioned compound of formula I or the pharmaceutically acceptable form thereof is hydrogen, halogen, or C₁₋₄ alkyl, and the C₁₋₄ alkyl is optionally substituted with one or more substituents selected from halogen, C₁₋₄ alkyl, -(C₁₋₄ alkylene)-O-(C₁₋₄ alkyl), or -(C₁₋₄ alkylene)-O-C(=O)-(C₁₋₄ alkyl).

In some embodiments, R⁵ in the above-mentioned compound of formula I or the pharmaceutically acceptable form thereof is hydrogen, halogen, or C₁₋₄ alkyl, and the C₁₋₄ alkyl is optionally substituted with one or more substituents selected from halogen, C₁₋₄ alkyl, -CH₂-O-CH₃, or -CH₂-O-C(=O)-CH₃.

In some preferred embodiments, R⁵ in the above-mentioned compound of formula I or the pharmaceutically acceptable form thereof is hydrogen, halogen, or methyl.

In some embodiments, L¹ in the above-mentioned compound of formula I or the pharmaceutically acceptable form thereof is -NR⁶C(=O)-, -NR⁶S(=O)₂-, -C(=O)NR⁶-, or - S(=O)₂NR⁶-, and each R⁶ is independently hydrogen, C₁₋₄ alkyl, or C₃₋₆ cycloalkyl.

In some preferred embodiments, L¹ in the above-mentioned compound of formula I or the pharmaceutically acceptable form thereof is -NR⁶C(=O)-, -NR⁶S(=O)₂-, -C(=O)NR⁶-, or - S(=O)₂NR⁶-, and each R⁶ is independently hydrogen or C₁₋₄ alkyl.

In some more preferred embodiments, L¹ in the above-mentioned compound of formula I or the pharmaceutically acceptable form thereof is -NHC(=O)-, -N(CH₃)-C(=O)-, -NHS(=O)₂-, -C(=O)NH-, or -S(=O)₂NH-.

In some embodiments, ring A in the above-mentioned compound of formula I or the pharmaceutically acceptable form thereof is a 5-10 membered heteroaryl, ring A is optionally substituted with one or more R⁷; and each R⁷ is independently halogen, C₁₋₄ alkyl, or C₁₋₄ haloalkyl.

In some preferred embodiments, ring A in the above-mentioned compound of formula I or the pharmaceutically acceptable form thereof is pyrrolyl, pyrazolyl, imidazolyl, thiazolyl, or oxazolyl, ring A is optionally substituted with one or more R⁷, and each R⁷ is independently halogen, methyl, ethyl, or trifluoromethyl.

In some more preferred embodiments, ring A in the above-mentioned compound of formula I or the pharmaceutically acceptable form thereof is pyrrolyl or imidazolyl, ring A is optionally substituted with one or more R⁷, and each R⁷ is independently halogen or methyl.

In some particularly preferred embodiments, ring A in the above-mentioned compound of formula I or the pharmaceutically acceptable form thereof is 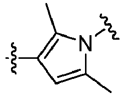 or

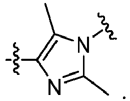

In some embodiments, L² in the above-mentioned compound of formula I or the pharmaceutically acceptable form thereof is a single bond, or CR⁸R⁹; and R⁸ and R⁹ are each independently hydrogen, halogen, or C₁₋₄ alkyl.

In some preferred embodiments, L² in the above-mentioned compound of formula I or the pharmaceutically acceptable form thereof is a single bond, -CH₂- or -CH(CH₃)-.

In some embodiments, ring B in the above-mentioned compound of formula I or the pharmaceutically acceptable form thereof is C₆₋₁₀ aryl or a 5-12 membered heteroaryl, ring B is optionally substituted with one or more R¹⁰, and each R¹⁰ is independently halogen, cyano, amino, nitro, hydroxyl, C₁₋₆ alkyl, C₁₋₆ haloalkyl, C₁₋₆ alkoxy, C₁₋₆ haloalkoxy, C₃₋₆ cycloalkyl, or - C(=O)-NH₂.

In some preferred embodiments, ring B in the above-mentioned compound of formula I or the pharmaceutically acceptable form thereof is C₆₋₁₀ aryl or a 5-10 membered heteroaryl, ring B is optionally substituted with one or more R¹⁰, and each R¹⁰ is independently halogen, cyano, amino, nitro, hydroxyl, C₁₋₄ alkyl, C₁₋₄ alkoxy, or -C(=O)-NH₂.

In some more preferred embodiments, ring B in the above-mentioned compound of formula I or the pharmaceutically acceptable form thereof is phenyl, pyrazinyl, pyridyl, pyrimidinyl, furanyl, oxazolyl, thienyl, thiazolyl, pyrazolyl, or imidazolyl, ring B is optionally substituted with one or more R¹⁰, and each R¹⁰ is independently halogen, cyano, amino, nitro, hydroxyl, methyl, or -C(=O)-NH₂.

In some particularly preferred embodiments, ring B in the above-mentioned compound of formula I or the pharmaceutically acceptable form thereof is

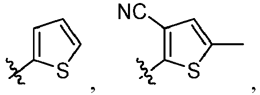

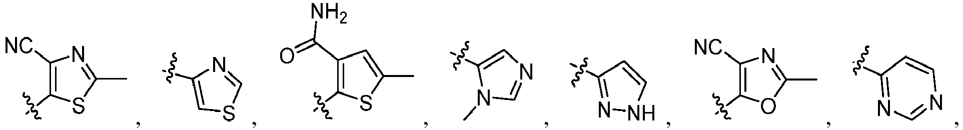

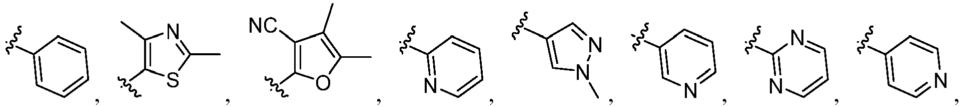

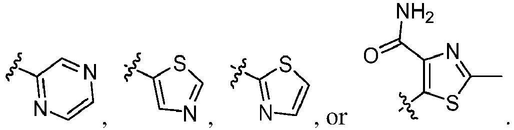

In some embodiments, the above-mentioned compound of formula I or the pharmaceutically acceptable form thereof is a compound having the structure of any one of formula I-1, formula I-2, formula I-3, formula I-4, formula I-5, formula I-6, formula I-19 or formula I-20 or a pharmaceutically acceptable form thereof: 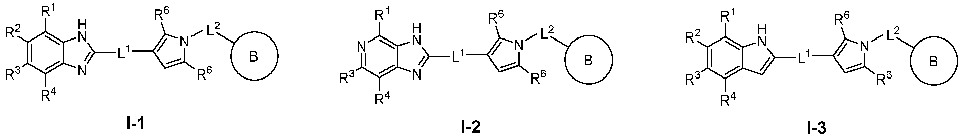 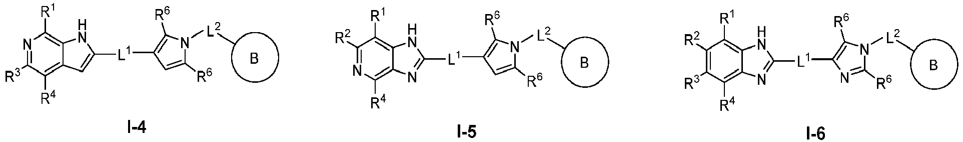 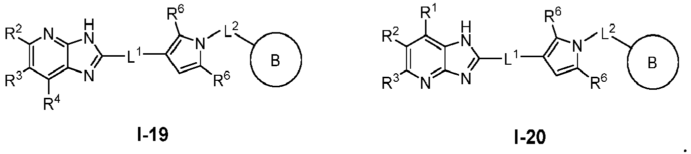 wherein R¹, R², R³, R⁴, R⁶, L¹, L² and ring B are as defined in formula I.

In some preferred embodiments, the above-mentioned compound of formula I or the pharmaceutically acceptable form thereof is a compound having the structure of any one of formula I-7, formula I-8, formula I-9, formula I-10, formula I-11, formula I-12, formula I-13, formula I-14, formula I-15, formula I-16, formula I-17, formula I-18, formula I-21 or formula I-22 or a pharmaceutically acceptable form thereof: 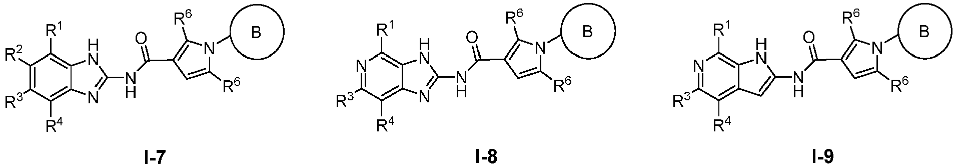 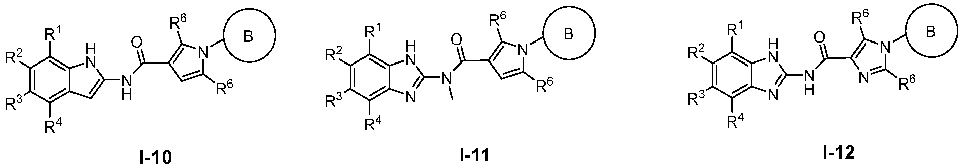 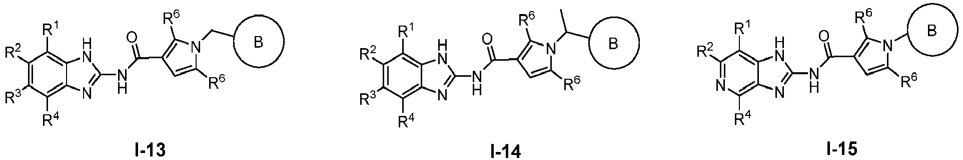 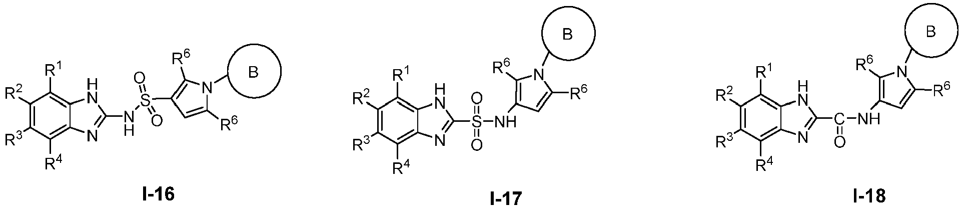 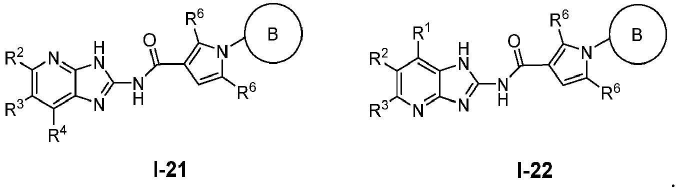 wherein R¹, R², R³, R⁴, R⁶, and ring B are as defined in formula I.

It should be understood by those skilled in the art that the present disclosure encompasses compounds derived from any combination of various embodiments. Embodiments obtained by combining a technical feature or a preferred technical feature in one embodiment with a technical feature or a preferred technical feature in another embodiment are also included within the scope of the present disclosure.

In a second aspect, the present disclosure also provides the following compounds or the pharmaceutically acceptable salts, esters, stereoisomers, tautomers, solvates, N-oxides, isotopically labeled forms, metabolites or prodrugs thereof:

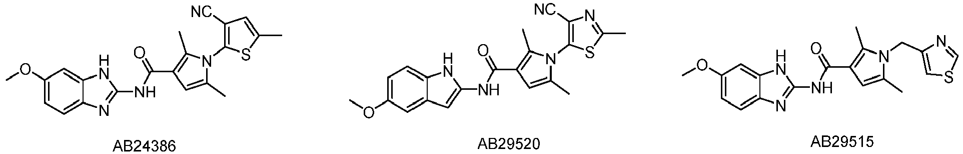

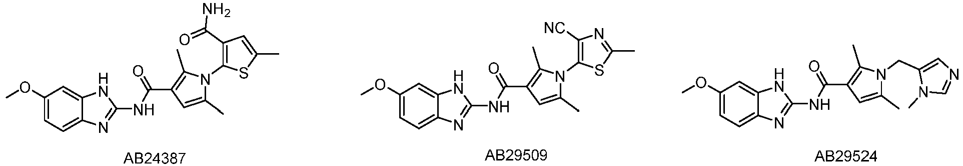

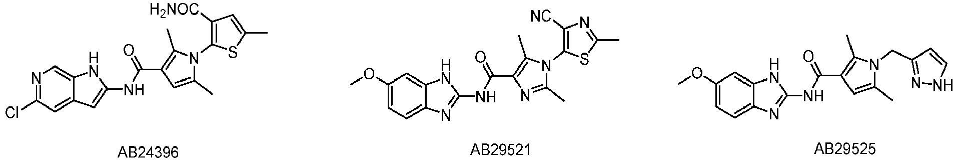

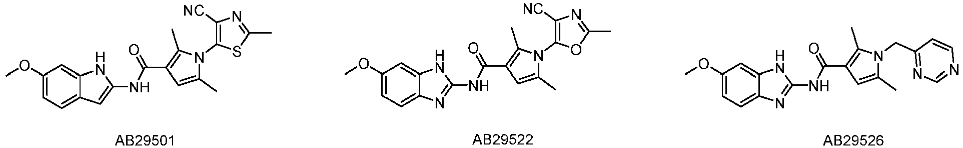

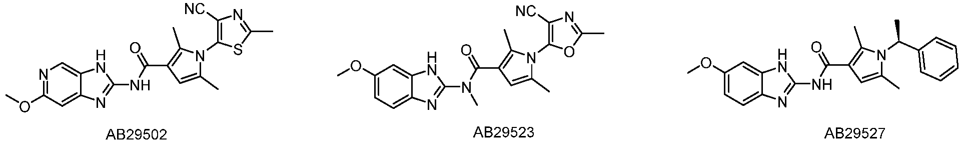

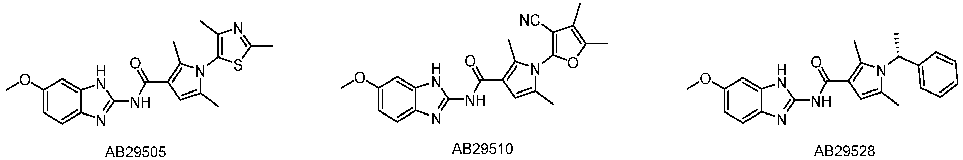

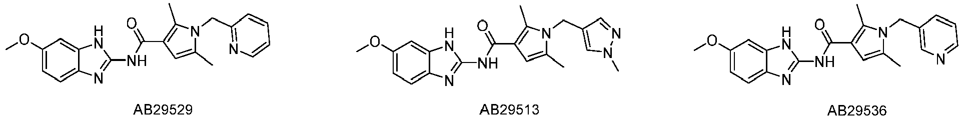

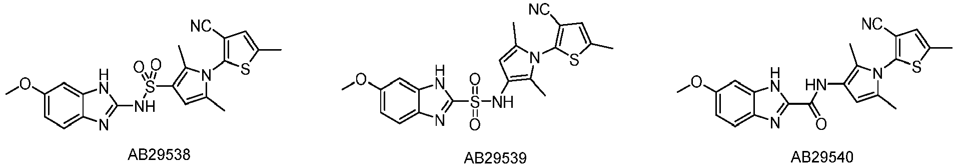

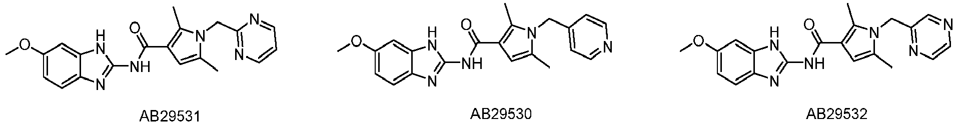

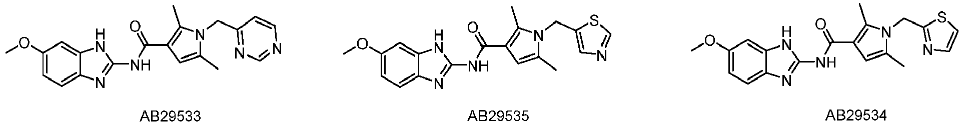

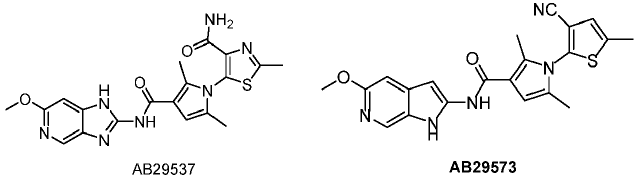

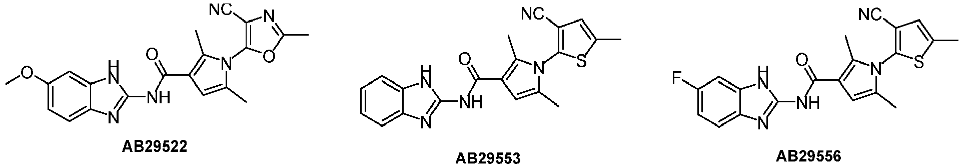

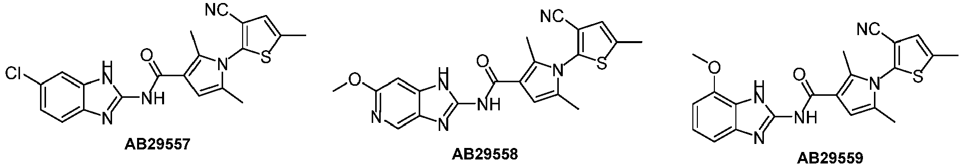

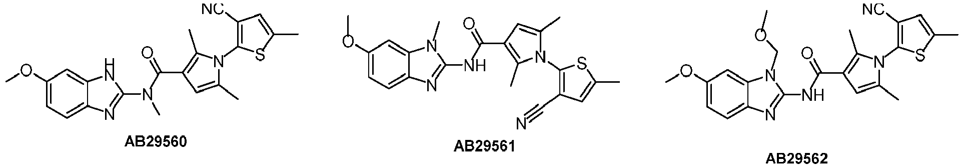

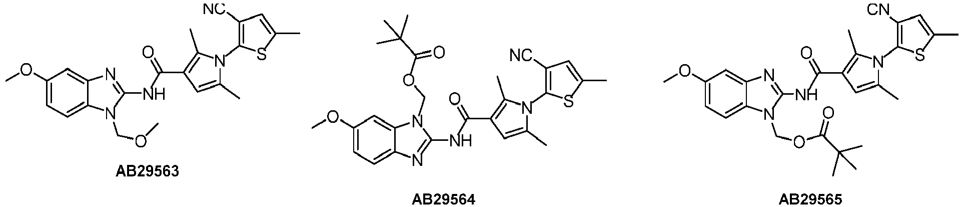

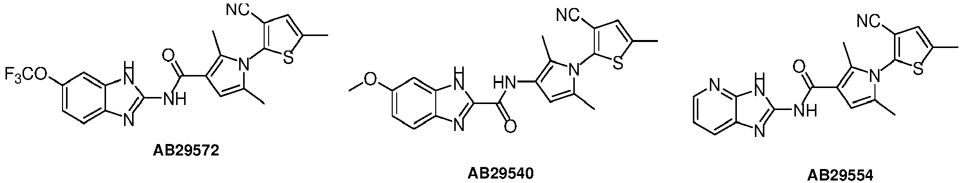

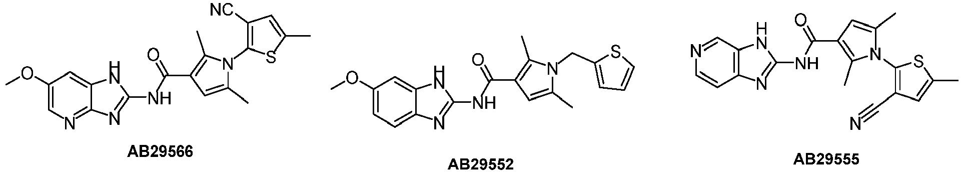

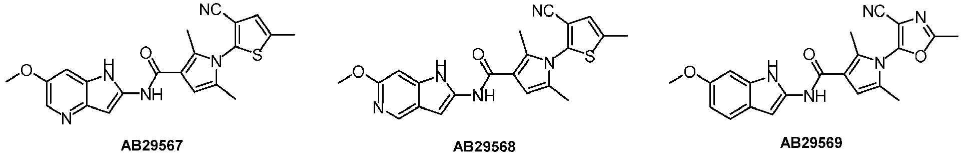

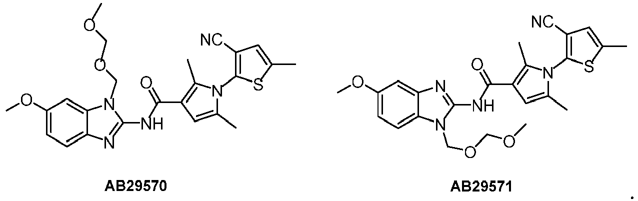

In a third aspect, the present disclosure provides a pharmaceutical composition, comprising at least one of the above-mentioned compounds or the pharmaceutically acceptable form thereof, and one or more pharmaceutically acceptable carriers.

In a fourth aspect, the present disclosure provides the above-mentioned compounds or the pharmaceutically acceptable form thereof or the above-mentioned pharmaceutical composition, for use as a DHX33 inhibitor for preventing and/or treating a disease or a disorder at least partially mediated by DHX33.

In a fifth aspect, the present disclosure provides the use of the above-mentioned compounds or the pharmaceutically acceptable form thereof or the above-mentioned pharmaceutical composition in preparation of a drug for preventing and/or treating a disease or a disorder at least partially mediated by DHX33.

In a sixth aspect, the present disclosure provides a method for preventing and/or treating a disease or a disorder at least partially mediated by DHX33, comprising the following step of administering a prophylactically and/or a therapeutically effective amount of the above-mentioned compounds or the pharmaceutically acceptable form thereof or the above-mentioned pharmaceutical composition to an individual in need thereof.

The present disclosure is not limited to the specific embodiments described herein. It should also be understood that the terms used herein are merely used for describing rather than limiting specific embodiments.

### Definition of Terms

Unless otherwise specified, the meanings of the following terms in the present disclosure are as follows.

The terms "comprise", "include", "have" or "contain" or any other variation thereof are intended to encompass non-exclusive or open-ended inclusions. For example, a composition, method or device comprising a series of elements is not necessarily limited to the elements that have been explicitly listed, and may also comprise other elements that are not explicitly listed or the elements inherent to the above-described composition, method or device.

When the lower limit and the upper limit of a numerical range are disclosed, any numerical value or any subrange falling within this range is intended to be specifically disclosed. In particular, each numerical range (for example, in the form of "about a to b", or equivalent form of "approximately a to b", or equivalent form of "about a-b") of a parameter disclosed herein should be understood to encompass each numerical value and each subrange therein. For example, "C₁₋₄" should be understood to encompass any subrange and each point value therein, e.g., C₂₋₄, C₃₋₄, C₁₋₂, C₁₋₃, C₁₋₄ and the like, as well as C₁, C₂, C₃, C₄ and the like. For another example, "5-10 membered" should be understood to encompass any subrange and each point value therein, e.g., 5-6 membered, 5-7 membered, 5-8 membered, 5-9 membered, 6-7 membered, 6-8 membered and the like, as well as 5 membered, 6 membered, 7 membered, 8 membered, 9 membered, 10 membered and the like.

The term "pharmaceutical composition" refers to a composition that may be used as a drug and comprises a pharmaceutically active ingredient (or a therapeutic agent) and alternatively one or more pharmaceutically acceptable carriers. The term "pharmaceutically acceptable carrier" refers to an excipient that is administered together with a therapeutic agent and is suitable for contacting with the tissues of human beings and/or other animals without excessive toxicity, irritation, allergic response or other problems or complications corresponding to a reasonable benefit/risk ratio within the scope of reasonable medical judgment. Pharmaceutically acceptable carriers usable in the present disclosure include but are not limited to: a) diluents; b) lubricants; c) binders; d) disintegrants; e) absorbing agents, colorants, flavoring agents and/or sweeteners; f) emulsifying agents or dispersing agents; and/or g) substances that enhance the absorption of the compound, and the like.

The above-mentioned pharmaceutical composition may act systemically and/or locally. For this purpose, they may be administered via suitable routes such as parenteral route, topical route, intravenous route, oral route, subcutaneous route, intraarterial route, intradermal route, transdermal route, rectal route, intracranial route, intraperitoneal route, intranasal route, intramuscular route, or may be administered as an inhalant.

The administration via the above-mentioned routes may be realized via suitable dosage forms. Dosage forms usable in the present disclosure include but are not limited to: tablets, capsules, lozenges, hard candies, powders, sprays, creams, ointments, suppositories, gels, pastes, lotions, ointments, aqueous suspensions, injectable solutions, elixirs, syrups, and the like.

When administered orally, the above-mentioned pharmaceutical composition may be prepared into any orally acceptable formulation, including but not limited to tablets, capsules, aqueous solutions, aqueous suspensions, and the like.

The above-mentioned pharmaceutical composition may also be administered in the form of sterile injection, including sterile injectable aqueous suspensions or oily suspensions, or sterile injectable aqueous solutions or oily solutions. Among these, usable carriers include but are not limited to water, Ringer's solution and isotonic sodium chloride solution. In addition, sterile non-volatile oils such as monoglycerides or diglycerides may also be used as a solvent or a suspending medium.

The above-mentioned pharmaceutical composition may comprise 0.01 mg to 1000 mg of at least one of the above-mentioned compounds or a pharmaceutically acceptable form thereof.

The term "a disease or a disorder at least partially mediated by DHX33" refers to a disease (such as cancer, viral infection and inflammation) of which the pathogeneses at least include DHX33-related factors in part.

The term "effective amount" refers to a dosage that is capable of inducing a cell, a tissue, an organ or an organism (e.g., an individual) to generate biological or medical response and is sufficient to achieve the desired prophylactic and/or therapeutic effects.

Dosing regimens may be adjusted to provide the optimal desired response. For example, the drug may be administered in a single dose, may be administered in divided doses over time, or may be administered in a dose that is reduced or increased proportionately according to the actual situation. It could be understood that, for any particular individual, the specific dosing regimen should be adjusted as needed and according to the professional judgment of the person administering the composition or supervising the administration of the composition.

The term "in need thereof' refers to a judgment of a physician or other nursing staff that an individual needs or will benefit from the prophylactic and/or therapeutic process, and this judgment is obtained based on various factors in the field of expertise of the physician or other nursing staff.

The term "individual" (or referred to as subject) refers to a human or a non-human animal. The individuals of the present disclosure include individuals suffering from a disease and/or disorder (patients) and normal individuals. The non-human animals of the present disclosure include all vertebrates, for example, non-mammals such as birds, amphibians and reptiles, and mammals such as non-human primates, livestock and/or domesticated animals (such as sheep, dogs, cats, cows and pigs).

The term "treating" means alleviating or eliminating the targeted disease or disorder. If a subject receives a therapeutic amount of the compound or the pharmaceutically acceptable form thereof of the present disclosure or the pharmaceutical composition of the present disclosure and this subject exhibits observable and/or detectable remission and/or improvement in at least one indicator or symptom, it indicates that this subject has been successfully "treated". It could be understood that treatment not only includes the complete treatment, but also includes a case where the complete treatment has not been achieved while some biologically or medically relevant results have been achieved. To be specific, "treating" means that the compound or the pharmaceutically acceptable form thereof of the present disclosure or the pharmaceutical composition of the present disclosure is capable of realizing at least one of the following effects, for example, (1) preventing the occurrence of a disease in an animal that may be predisposed to the disease but have not yet experienced or exhibited the pathology or symptomatology of the disease, (2) inhibiting a disease (that is, preventing further progression of pathology and/or symptomatology) in an animal that is experiencing or exhibiting the pathology or symptomatology of the disease, and (3) ameliorating a disease (that is, reversing pathology and/or symptomatology) in an animal that is experiencing or exhibiting the pathology or symptomatology of the disease.

The term "pharmaceutically acceptable salt" refers to a salt of a compound of the present disclosure which is substantially non-toxic to an organism. Pharmaceutically acceptable salts generally include but are not limited to salts formed by the reaction of the compounds of the present disclosure with pharmaceutically acceptable inorganic/organic acids or inorganic/organic bases, and such salts are also referred to as acid addition salts or base addition salts. As for reviews of suitable salts, please refer to, for example, Jusiak, Soczewinski, et al., Remington's Pharmaceutical Sciences [M], Mack Publishing Company, 2005 and Stahl, Wermuth, Handbook of Pharmaceutical Salts: Properties, Selection, and Use [M], Wiley-VCH, 2002**.** A method for preparing the pharmaceutically acceptable salts of the compounds of the present disclosure is known to those skilled in the art.

The term "pharmaceutically acceptable ester" refers to an ester that is substantially non-toxic to an organism and is hydrolyzed to a compound of the present disclosure or a salt thereof in the organism. Pharmaceutically acceptable esters generally include but are not limited to esters formed by the compounds of the present disclosure and pharmaceutically acceptable carboxylic acids or sulfonic acids, and such esters are also referred to as carboxylic acid esters or sulfonic acid esters.

The term "isomers" refers to compounds that have the same molecular weight due to the same number and type of atoms while having different spatial arrangement of atoms or configurations.

The term "stereoisomer" (or referred to as "optical isomer") refers to a stable isomer that has a vertical asymmetric plane resulting from at least one chiral factor (including a chiral center, a chiral axis, a chiral plane, and the like) and thus is capable of enabling the rotation of the plane-polarized light. Since asymmetric center(s) and other chemical structures that may result in stereoisomerism exist in the compounds of the present disclosure, the present disclosure also includes these stereoisomers and the mixtures thereof. Unless otherwise indicated, all stereoisomeric forms of the compounds of the present disclosure are within the scope of the present disclosure.

The term "tautomers" (or referred to as "tautomeric forms") refers to structural isomers that have different energy and are interconvertible via a low energy barrier. If tautomerism is possible (for example, in solution), a chemical equilibrium of tautomers may be achieved. For example, proton tautomers (or referred to as proton-transfer tautomers) include but are not limited to those obtained by the interconversion via proton transfer such as keto-enol tautomerism, imine-enamine tautomerism and amide-iminol tautomerism. Unless otherwise indicated, all tautomeric forms of the compounds of the present disclosure are within the scope of the present disclosure.

The term "solvate" refers to a substance formed by the association of a compound of the present disclosure (or a pharmaceutically acceptable salt thereof) with at least one kind of solvent molecule via non-covalent intermolecular force. For example, solvates include but are not limited to hydrates (including hemihydrates, monohydrates, dihydrates, trihydrates, and the like), ethanolates, acetonates, and the like.

The term "N-oxide" refers to a compound formed by the oxidation of the nitrogen atom(s) in the structure of a tertiary amine-based compound or a nitrogen-containing (aromatic) heterocyclic compound. For example, the nitrogen atoms in the parent structure of the above-mentioned compound may be oxidized, so that the corresponding N-oxide may be formed.

The term "isotopically labeled form" refers to a derivative compound formed by replacing a particular atom in a compound of the present disclosure with an isotope thereof. Unless otherwise indicated, the compounds of the present disclosure comprise various isotopes of H, C, N, O, F, P, S and C1, such as, but not limited to, ²H(D), ³H(T), ¹³C, ¹⁴C, ¹⁵N, ¹⁷O, ¹⁸O, ¹⁸F, ³¹P, ³²P, ³⁵S, ³⁶S and ³⁷Cl.

The term "metabolite" refers to a derivative compound formed by the metabolism of a compound of the present disclosure. As for further information on metabolism, please refer to Goodman and Gilman's: The Pharmacological Basis of Therapeutics (9th ed.) [M], McGraw-Hill International Editions, 1996. The present disclosure encompasses all possible forms of the metabolites of the compounds of the present disclosure, that is, substances formed in an individual to whom the compound(s) of the present disclosure is administered. The metabolites of compounds may be identified by techniques well known in the art, and the activity thereof may be characterized by assays.

The term "prodrug" refers to a derivative compound capable of directly or indirectly providing a compound of the present disclosure upon administration to an individual. Particularly preferred derivative compounds or prodrugs are compounds that are capable of improving the bioavailability of the compounds of the present disclosure (e.g., enabling easier absorption into blood) or facilitating the delivery of the parent compounds to the sites of action (e.g., the lymphatic system) when administered to an individual. Unless otherwise indicated, all forms of the prodrugs of the compounds of the present disclosure are within the scope of the present disclosure, and various forms of the prodrugs are known in the art, for example, see T. Higuchi, V. Stella, Pro-drugs as Novel Drug Delivery Systems [J], American Chemical Society, Vol. 14, 1975. In addition, the present disclosure also encompasses the compounds of the present disclosure that contain protecting groups. In any process of preparing the compounds of the present disclosure, it may be essential and/or desirable to protect the sensitive group(s) or reactive group(s) on any relevant molecule, and the chemically protected forms of the compounds of the present disclosure are thus formed. It could be achieved by conventional protecting groups, such as the protecting groups described in T. W. Greene, P. G. M. Wuts, Protective Groups in Organic Synthesis [M], John Wiley & Sons, 2006**.** These protecting groups may be removed at an appropriate subsequent stage using methods known in the art.

The term "each independently" means that at least two groups (or cyclic systems) that are present in the structure and have the same or similar range of values may have the same or different meanings under certain circumstances. For example, substituent X and substituent Y are each independently hydrogen, halogen, hydroxyl, cyano, alkyl or aryl, then when substituent X is hydrogen, substituent Y may be hydrogen, and may also be halogen, hydroxyl, cyano, alkyl or aryl; similarly, when substituent Y is hydrogen, substituent X may be hydrogen, and may also be halogen, hydroxyl, cyano, alkyl or aryl.

When used herein alone or in combination with other groups, the term "halogen" refers to fluorine (F), chlorine (Cl), bromine (Br) and iodine (I).

When used herein alone or in combination with other groups, the term "alkyl" refers to a linear or branched aliphatic hydrocarbon group. For example, the term "C₁₋₆ alkyl" used in the present disclosure refers to an alkyl group having 1 to 6 carbon atoms. For example, alkyl groups include but are not limited to methyl, ethyl, n-propyl, isopropyl, n-butyl, sec-butyl, isobutyl, tert-butyl, or the like. Alkyl groups may be optionally substituted or unsubstituted.

When used herein alone or in combination with other groups, the term "alkylene" refers to a linear or branched divalent saturated aliphatic hydrocarbon group, and the two groups (or fragments) attached to alkylene may be attached to either the same carbon atom or different carbon atoms. For example, the term "C₁₋₆ alkylene" used herein refers to an alkylene group having 1 to 6 carbon atoms (e.g., methylene, 1,1-ethylene, 1,2-ethylene, 1,2-propylene, 1,3-butylene, and the like). Alkylene groups may be optionally substituted or unsubstituted.

When used herein alone or in combination with other groups, the term "haloalkyl" refers to an alkyl group substituted with one or more (such as 1 to 3) same or different halogen atoms. For example, the term "C₁₋₆ haloalkyl" used in the present disclosure refers to a haloalkyl group having 1 to 6 carbon atoms. For example, haloalkyl groups include but are not limited to -CH₂F, -CHF₂, -CF₃, -CH₂CF₃, -CF₂CF₃, -CH₂CH₂CF₃, -CH₂Cl, and the like. Haloalkyl groups may be optionally substituted or unsubstituted.

When used herein alone or in combination with other groups, the term "hydroxyalkyl" refers to an alkyl group substituted with one or more (such as 1 to 3) hydroxyl groups. For example, the term "C₁₋₆ hydroxyalkyl" used in the present disclosure refers to a hydroxyalkyl group having 1 to 6 carbon atoms. For example, hydroxyalkyl groups include but are not limited to 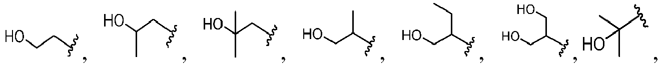 or the like. Hydroxyalkyl groups may be optionally substituted or unsubstituted.

When used herein alone or in combination with other groups, the term "alkoxy" refers to an alkyl group that is attached to the remaining part of the molecule via an oxygen atom. For example, alkoxy groups include but are not limited to methoxy, ethoxy, n-propoxy, isopropoxy, n-butoxy, isobutoxy, tert-butoxy, and the like. Alkoxy groups may be optionally substituted or unsubstituted.

When used herein alone or in combination with other groups, the term "haloalkoxy" refers to a monovalent linear or branched haloalkyl-O-group, which is substituted with at least one atom selected from fluorine, chlorine, bromine and iodine, may comprise degree(s) of unsaturation, and is attached to other groups via a single bond attached to an oxygen atom, such as C₁₋₆ haloalkoxy. For example, haloalkoxy groups include but are not limited to fluoromethoxy (-OCH₂F), difluoromethoxy (-OCHF₂), trifluoromethoxy(-OCF₃), 1-fluoroethoxy (-OCHFCH₃), 2-fluoroethoxy (-OCH₂CH₂F), 1,2-difluoroethoxy (-OCHFCH₂F), 2,2-difluoroethoxy (-OCH₂CHF₂), 1,2,2-trifluoroethoxy (-OCHFCHF₂), 2,2,2-trifluoroethoxy (-OCH₂CF₃), and the like.

When used herein alone or in combination with other groups, the term "cycloalkyl" refers to a monocyclic or polycyclic (such as bicyclic) non-aromatic hydrocarbon group that is saturated or partially saturated. For example, the term "C₃₋₆ cycloalkyl" used in the present disclosure refers to a cycloalkyl group having 3 to 6 carbon atoms. For example, cycloalkyl groups include but are not limited to cyclopropyl, cyclobutyl, cyclopentyl, cyclohexyl, or the like. Cycloalkyl groups may be optionally substituted or unsubstituted.

When used herein alone or in combination with other groups, the term "heterocyclyl" refers to a monocyclic or polycyclic (for example, bicyclic such as fused cyclic, bridged cyclic or spiro cyclic) non-aromatic group that is saturated or partially saturated and has ring atoms consisting of carbon atoms and at least one heteroatom selected from N, O and S, wherein the sulfur atom is optionally substituted to form S(=O), S(=O)₂ or S(=O)(=NR^{x}) and R^{x} is independently H or C₁₋₄ alkyl. A heterocyclyl group may be attached to the remaining part of the molecule via any one of the ring atoms if the requirements of valence are satisfied. For example, the term "3-8 membered heterocyclyl" used in the present disclosure refers to a heterocyclic group having 3 to 8 ring atoms. Common heterocyclyl groups include (but are not limited to) oxiranyl, aziridinyl, azetidinyl, oxetanyl, tetrahydrofuranyl, dioxolyl, pyrrolidinyl, pyrrolidonyl, imidazolidinyl, pyrazolidinyl, tetrahydropyranyl, piperidinyl, piperazinyl, morpholinyl, thiomorpholinyl, dithianyl or trithianyl. A heterocyclic group in the present disclosure is optionally substituted with one or more substituents as described in the present disclosure.

When used herein alone or in combination with other groups, the term "aryl" refers to a monocyclic or fused polycyclic aromatic hydrocarbon group that has a conjugated system of π electrons. For example, the term "C₆₋₁₀ aryl" used in the present disclosure refers to an aryl group having 6 to 10 carbon atoms. Common aryl group include (but are not limited to) phenyl, naphthyl, anthryl, phenanthryl, acenaphthenyl, azulenyl, fluorenyl, indenyl, pyrenyl, and the like. An aryl group in the present disclosure is optionally substituted with one or more substituents as described in the present disclosure.

When used herein alone or in combination with other groups, the term "heteroaryl" refers to a monocyclic or fused polycyclic aromatic group that has a conjugated system of π electrons and has ring atoms consisting of carbon atoms and at least one heteroatom selected from N, O and S. A heteroaryl group may be attached to the remaining part of the molecule via any one of the ring atoms if the requirements of valence are satisfied. For example, the term "5-10 membered heteroaryl" used in the present disclosure refers to a heteroaryl group having 5 to 10 ring atoms. Common heteroaryl groups include (but are not limited to) thienyl, furanyl, pyrrolyl, oxazolyl, thiazolyl, imidazolyl, pyrazolyl, isoxazolyl, isothiazolyl, oxadiazolyl, triazolyl, thiadiazolyl, pyridyl, pyridazinyl, pyrimidinyl, pyrazinyl, triazinyl and the benzo derivatives thereof, pyrrolopyridyl, pyrrolopyrazinyl, pyrazolopyridyl, imidazolopyridyl, pyrrolopyrimidinyl, pyrazolopyrimidinyl, purinyl, and the like. A heteroaryl group in the present disclosure is optionally substituted with one or more substituents as described in the present disclosure (e.g., halogen, C₁₋₆ alkyl, and the like).

When used herein alone or in combination with other groups, the term "hydroxyl" refers to -OH.

When used herein alone or in combination with other groups, the term "cyano" refers to -CN.

When used herein alone or in combination with other groups, the term "amino" refers to -NH₂.

When used herein alone or in combination with other groups, the term "nitro" refers to -NO₂.

### BRIEF DESCRIPTION OF THE DRAWINGS

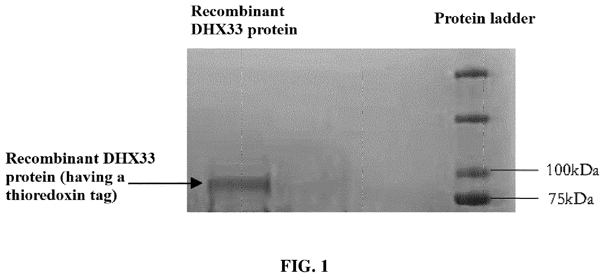

FIG. 1 shows the analysis of the results obtained after the SDS-PAGE separation and Coomassie brilliant blue staining of the recombinant DHX33 protein prepared by using the method of the present disclosure.

### DETAILED DESCRIPTION

In order to make the objects and technical solutions of the present disclosure more explicit, the embodiments of the present disclosure are described in detail below with reference to examples. However, those skilled in the art would understand that the following examples are merely for the illustration of the present disclosure and should not be construed as limiting the scope of the present disclosure.

The reagents or instruments used in the examples are all conventional products that are commercially available. If no specific conditions are specified, the routine conditions or the conditions suggested by the manufacturer shall be followed. The term "room temperature" used in the present disclosure refers to 20°C ± 5°C. When used for modifying certain numerical value or numerical range, the term "about" used in the present disclosure is intended to include this numerical value or numerical range as well as the error range (acceptable to those skilled in the art) of this numerical value or numerical range, for example, this error range is ±10%, ±5%, ±4%, ±3%, ±2%, ±1%, ±0.5%, and the like.

The structures of the compounds described in the following examples are determined by nuclear magnetic resonance (NMR) and/or mass spectrometry (MS).

A Bruker 400 MHz NMR spectrometer is used as the measurement instrument of nuclear magnetic resonance (NMR), the solvents used for determination are deuterated methanol (CD₃OD), deuterated chloroform (CDCl₃) and hexadeuterated dimethyl sulfoxide (DMSO-d₆), and the internal standard substance is tetramethylsilane (TMS). In ¹H NMR, some of the hydrogen atoms may not give rise to peaks due to interference from a salt or a solvent.

The meanings of the abbreviations in the nuclear magnetic resonance (NMR) data in the following examples are as follows.

s: singlet, d: doublet, t: triplet, q: quartet, dd: doublet of doublets, qd: quartet of doublets, ddd: doublet of doublet of doublets, ddt: doublet of doublet of triplets, dddd: doublet of doublet of doublet of doublets, m: multiplet, br: broad singlet, J: coupling constant, Hz: Hertz, δ : chemical shift.

All chemical shift (6) values are given in parts per million (ppm).

An Agilent 6120B mass spectrometer is used as the measurement instrument of mass spectrometry (MS), and the ion source is an electrospray ion source (ESI).

An Agilent 1200DAD high-pressure liquid chromatograph (chromatographic column: Sunfirc C18, 150 × 4.6 mm, 5 µm) and a Waters 2695-2996 high-pressure liquid chromatograph (chromatographic column: Gimini C18, 150 × 4.6 mm, 5 µm) are used for HPLC assay.

GF254 silica gel plates manufactured by Qingdao Marine Chemical Inc. are used as the silica gel plates for thin layer chromatography, the specification of the silica gel plates used for thin layer chromatography (TLC) is 0.15 mm to 0.2 mm, and the specification of the silica gel plates used for the separation and purification of the product via thin layer chromatography is 0.4 mm to 0.5 mm.

Silica gel (200 mesh to 300 mesh) manufactured by Qingdao Marine Chemical Inc. is generally used as the carrier in column chromatography.

Thin layer chromatography (TLC) is adopted to monitor the reaction progress in the examples. The solvent systems used in thin layer chromatography include (A) dichloromethane/methanol system and (B) petroleum ether/ethyl acetate system. The volume ratio of solvents is adjusted according to the polarity of the compound.

The eluent systems for column chromatography and the solvent systems for thin layer chromatography adopted for the purification of compounds include (A) dichloromethane/methanol system and (B) petroleum ether/ethyl acetate system. The volume ratio of solvents is adjusted according to the polarity of the compound, and a small amount of triethylamine or an acidic or basic reagent may also be added for adjustment.

### Synthesis of compounds

### Example 1: Synthesis of Compound AB29502 (1-(4-cyano-2-methylthiazol-5-yl)- N-(6-methoxy-3H-imidazo[4,5-c]pyridin-2-yl)-2,5-dimethyl-1H-pyrrole-3-carboxamide)

### (1) Preparation method of Compound 3 (ethyl 5-(2,5-dimethyl-1H-pyrrol-1-yl)- 2-methylthiazole-4-carboxylate)

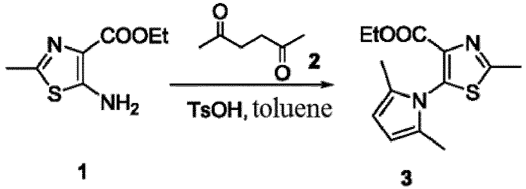

Compound **1** (ethyl 5-amino-2-methyl-1,3-thiazole-4-carboxylate) (5 g, 26.84 mmol, 1.0 eq) was dissolved in toluene (100 mL). Compound 2 (2,5-hexanedione) (4.6 g, 40.26 mmol, 1.5 eq), 3A molecular sieve (10 g) and p-toluenesulfonic acid (1.8 g, 10.73 mmol, 0.4 eq) were added thereto. The mixture was heated under reflux and stirred overnight. The solid was filtered and concentrated, and the residue was separated by flash column chromatography (petroleum ether/ethyl acetate = 10/1), so as to obtain Compound **3** (ethyl 5-(2,5-dimethyl-1H-pyrrol-1-yl)-2-methylthiazole-4-carboxylate) as a white solid (3 g, yield: 42.8%). MS (ESI) m/z: 265 [M+H]⁺. TLC: petroleum ether/ethyl acetate (5/1); R_{f} *(Compound 1*) = 0.2; R_{f} *(Compound 3*) = 0.5.

### (2) Preparation method of Compound 4 (5-(2,5-dimethyl-1H-pyrrol-1-yl)-2-methylthiazole-4-carboxamide)

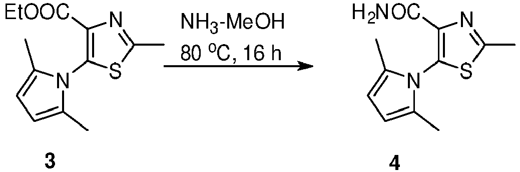

Compound **3** (ethyl 5-(2,5-dimethyl-1H-pyrrol-1-yl)-2-methylthiazole-4-carboxylate) (3.0 g, 11.4 mmol, 1.0 eq) was dissolved in a mixed solution of ammonia/methanol (40 mL). The mixture was sealed in a reaction tube and heated and stirred at 80°C for 16 hours. The solid was filtered so as to obtain Compound **4** (5-(2,5-dimethyl-1H-pyrrol-1-yl)-2- methylthiazole-4-carboxamide) as a white solid (1.9 g, yield: 71.2%). MS (ESI) m/z: 236 [M+H]⁺. TLC: petroleum ether/ethyl acetate (3/1); R_{f} *(Compound 3*) = 0.6; R_{f} (*Compound 4*) = 0.4.

### (3) Preparation method of Compound 5 (5-(3-formyl-2,5-dimethyl-1H-pyrrol-1-yl)-2-methylthiazole-4-carbonitrile)

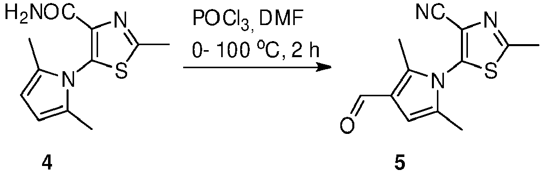

Phosphorus oxychloride (2.48 g, 16.2 mmol, 2.0 eq) was added dropwise to dimethylformamide (30 mL) at 0°C under the protection of nitrogen gas. The mixture was stirred at 0°C for 30 minutes and then warmed to room temperature. Compound **4** (5-(2,5-dimethyl-1H-pyrrol-1-yl)-2-methylthiazole-4-carboxamide) (1.9 g, 8.09 mmol, 1.0 eq) in dimethylformamide (4 mL) was added to the above-mentioned reaction system. Afterwards, the above-mentioned reaction solution was heated to 100°C and stirred for one hour under the protection of nitrogen gas. After the reaction solution was cooled, the mixture was poured into ice water and the pH of the resulting mixture was then adjusted to 10 with 30% NaOH aqueous solution. The mixture was extracted with ethyl acetate and washed with brine. Afterwards, the organic layer was dried over anhydrous sodium sulfate and concentrated. The residue was purified by flash column chromatography (petroleum ether/ethyl acetate = 2/1) so as to obtain Compound **5** (5-(3-formyl-2,5-dimethyl-1H-pyrrol-1-yl)-2-methylthiazole-4-carbonitrile) as a white solid (1.3 g, yield: 65.6%). MS (ESI) m/z: 246 [M+H]⁺. TLC: petroleum ether/ethyl acetate (2/1); R_{f} (*Compound 4*) = 0.3; R_{f} *(Compound 5*) = *0.5.*

### (4) Preparation method of Compound 6 (1-(4-cyano-2-methylthiazol-5-yl)-2,5-dimethyl-1H-pyrrole-3-carboxylic acid)

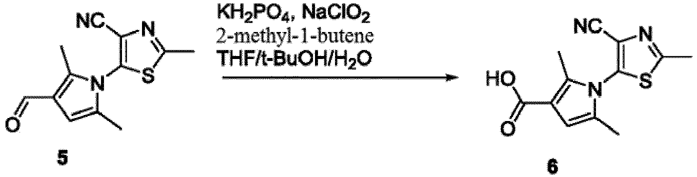

Compound **5** (5-(3-formyl-2,5-dimethyl-1H-pyrrol-1-yl)-2-methylthiazole-4-carbonitrile) (1.7 g, 6.93 mmol, 1.0 eq) was dissolved in tetrahydrofuran/tert-butanol/water (1/1/1, 45 mL). Potassium dihydrogen phosphate (4.71 g, 34.65 mmol, 5.0 eq), 2-methyl-1-butene (4.86 g, 69.3 mmol, 10.0 eq) and sodium chlorite (3.76 g, 41.58 mmol, 6.0 eq) were added thereto at 0°C. The reactants were stirred and refluxed at room temperature for 16 hours. After the completion of the reaction, the mixture was concentrated and then purified by preparative high-pressure liquid chromatography so as to obtain Compound **6** (1-(4-cyano-2-methylthiazol-5-yl)-2,5- dimethyl-1H-pyrrole-3-carboxylic acid) as a white solid (450 mg, yield: 25%). MS (ESI) m/z: 262 [M+H⁺].

### (5) Preparation method of Compound AB29502 (1-(4-cyano-2-methylthiazol-5-yl)-N-(6-methoxy-3H-imidazo[4,5-c]pyridin-2-yl)-2,5-dimethyl-1H-pyrrole-3-carboxamide)

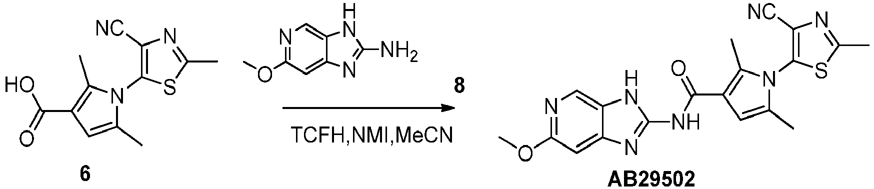

Compound **6** (1-(4-cyano-2-methylthiazol-5-yl)-2,5-dimethyl-1H-pyrrole-3-carboxylic acid) (55 mg, 0.21 mmol, 1.0 eq) was dissolved in acetonitrile (2.0 mL). Compound **8** (6-methoxy-3H-imidazo[4,5-c]pyridin-2-amine) (46 mg, 0.273 mmol, 1.3 eq), 2-methylimidazole NMI (69 mg, 0.84 mmol, 4.0 eq) and tetramethylchloroformamidinium hexafluorophosphate TCFH (71 mg, 0.25 mmol, 1.2 eq) were added thereto. The mixture was stirred at room temperature for 1 hour. After the completion of the reaction, the mixture was concentrated and then separated and purified by preparative high-pressure liquid chromatography, so as to obtain Compound **AB29502** (1-(4-cyano-2-methylthiazol-5-yl)-N-(6- methoxy-3H-imidazo[4,5-c]pyridin-2-yl)-2,5-dimethyl-1H-pyrrole-3-carboxamide) as a yellow solid (2.5 mg, yield: 2.9%). MS (ESI) m/z: 408.3 [M+H⁺]. ¹H NMR (400 MHz, CD₃OD-*d*4) δ 8.33 (s, 1H), 7.21 (s, 1H), 6.70 (s, 1H), 4.09 (s, 3H), 2.78 (s, 3H), 2.48 (s, 3H), 2.15 (s, 3H).

### Example 2: Preparation method of Compound AB29509 (1-(4-cyano-2-methylthiazol-5-yl)-N-(6-methoxy-1H-benzo[d]imidazol-2-yl)-2,5-dimethyl-1H-pyrrole-3-carboxamide)

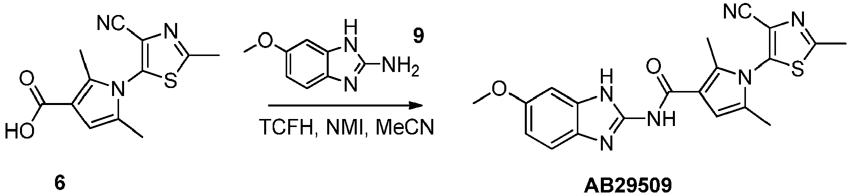

Compound **6** (1-(4-cyano-2-methylthiazol-5-yl)-2,5-dimethyl-1H-pyrrole-3-carboxylic acid) (100 mg, 0.38 mmol, 1.0 eq) was dissolved in acetonitrile (5.0 mL). Compound **9** (6-methoxy-1H-benzo[d]imidazole-2-amine)(81 mg, 0.50 mmol, 1.3 eq), 2-methylimidazole NMI (126 mg, 1.53 mmol, 4.0 eq) and tetramethylchloroformamidinium hexafluorophosphate TCFH (129 mg, 0.46 mmol, 1.2 eq) were added thereto. The mixture was stirred at room temperature for 1 hour. After the completion of the reaction, the mixture was concentrated and purified by preparative high-pressure liquid chromatography, so as to obtain Compound **AB29509** (1-(4-cyano-2-methylthiazol-5-yl)-N-(6-methoxy-1H-benzo[d]imidazol-2-yl)-2,5- dimethyl-1H-pyrrole-3-carboxamide) as a yellow solid (3.0 mg, yield: 1.9 %). MS (ESI) m/z: 407.10 [M+H⁺]. ¹H NMR (400 MHz, DMSO-*d*₆) δ 7.45 (d, *J* = 10 Hz, 1H), 7.10 (s, 1H), 6.88 (d, *J* = 8.8 Hz, 1H), 6.81 (s, 1H), 3.76 (s, 3H), 2.76 (s, 3H), 2.42 (s, 3H), 2.09 (s, 3H).

### Example 3: Synthesis of Compound AB24386 (1-(3-cyano-5-methylthiophen-2-yl)-N-(6-methoxy-1H-benzo[d]imidazol-2-yl)-2,5-dimethyl-1H-pyrrole-3-carboxamide)

### (1) Preparation method of Compound 3 (ethyl 2-acetyl-4-levulinate)

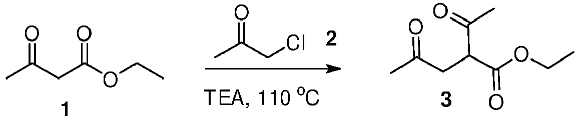

Compound **1** (ethyl acetoacetate) (5 g, 38.42 mmol, 1.0 eq) was dissolved in triethylamine (75 mL), and Compound **2** (chloroacetone) (3.5 g, 38.42 mmol, 1.0 eq) was added thereto. The reactants were reacted at 110°C for 2 hours under the protection of nitrogen gas. After being concentrated, the residue was dissolved in water (100 mL) and then extracted twice with dichloromethane (50 mL for each extraction). The organic layers were washed with brine, dried over anhydrous sodium sulfate, filtered and then concentrated. The residue was purified by flash column chromatography (petroleum ether/ethyl acetate = 100/1 to 50/1 to 20/1), so as to obtain Compound 3 (ethyl 2-acetyl-4-levulinate) as a colorless oil (1.3 g, yield: 18.3%). MS (ESI) m/z: 187 [M+H⁺]. TLC: PE/EA (2/1); R_{f} (Compound 1) = 0.6; R_{f} (Compound 3) = 0.4.

### (2) Preparation method of Compound 5 (ethyl 1-(3-cyano-5-methylthiophen-2-yl)- 2,5-dimethyl-1H-pyrrole-3-carboxylate)

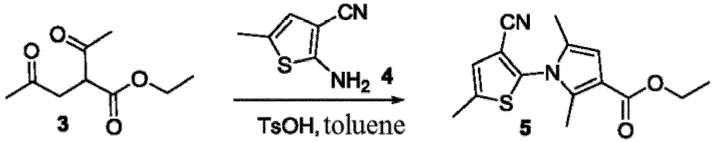

Compound **3** (ethyl 2-acetyl-4-levulinate) (1 g, 7.23 mmol, 1.0 eq) was dissolved in toluene (20 mL). Compound **4** (2-amino-3-cyano-5-methylthiophene) (1.6 g, 8.68 mmol, 1.2 eq) and p-toluenesulfonic acid (249 mg, 1.45 mmol, 0.2 eq) were added thereto. The reactants were stirred at 110°C for 16 hours. The solid was filtered and then concentrated. The residue was purified by flash column chromatography (petroleum ether/ethyl acetate = 50/1 to 30/1), so as to obtain Compound **5** (ethyl 1-(3-cyano-5-methylthiophen-2-yl)-2,5-dimethyl- 1H-pyrrole-3-carboxylate) as a yellow oil (860 mg, yield: 41%). MS (ESI) m/z: 289 [M+H⁺]. TLC: petroleum ether/ethyl acetate (10/1); R_{f} (*Compound 3*) = 0.2; R_{f} (*Compound 5*) = 0.4.

### (3) Preparation method of Compound AB24386 (1-(3-cyano-5-methylthiophen-2-yl)-N-(6-methoxy-1H-benzo[d]imidazol-2-yl)-2,5-dimethyl-1H-pyrrole-3-carboxamide)

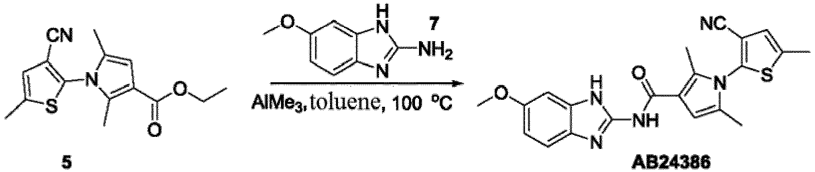

Trimethylaluminum (0.15 mL, 0.306 mmol, 1.0 eq, 2 M in toluene) was added into a mixture of Compound **5** (ethyl 1-(3-cyano-5-methylthiophen-2-yl)-2,5-dimethyl- 1H-pyrrole-3-carboxylate) (50 mg, 0.306 mmol, 1.0 eq) and Compound **7** (5-methoxy-1H-benzimidazole-2-amine) (88 mg, 0.306 mmol, 1.0 eq) that was dissolved in 1 mL of toluene. The reactants were stirred at 100°C for 16 hours. The mixture was cooled to room temperature and quenched with methanol (10 mL), and the pH of the resulting mixture was then adjusted to 3 with 3 M hydrochloric acid. The mixture was diluted with water (30 mL) and then extracted three times with ethyl acetate (20 ml for each extraction). The organic layers were washed with brine, dried over anhydrous sodium sulfate, filtered and then concentrated. The residue was purified by preparative high-pressure liquid chromatography Prep-HPLC (acetonitrile/water (containing 0.1% formic acid)) so as to obtain Compound **AB24386** (1-(3-cyano-5-methylthiophen-2-yl)-N-(6-methoxy-1H-benzo[d]imidazol-2-yl)-2,5-dimethyl -1H-pyrrole-3-carboxamide) as a white solid (5 mg, yield: 4 %). MS (ESI) m/z: 406 [M+H⁺]. ¹H NMR (400 MHz, DMSO-*d*₆): *δ* 12.04 (s, 1H), 11.20 (s, 1H), 7.32 (s, 1H), 7.29 (s, 1H), 6.98 (s, 1H), 6.89 (s, 1H), 6.69 (d, *J* = 7.2 Hz, 1H), 3.73 (s, 3H), 2.47 (s, 3H), 2.38 (s, 3H), 2.04 (s, 3H).

### Example 4: Preparation method of Compound AB24387 (1-(3-carbamoyl-5-methylthiophen-2-yl)-N-(6-methoxy-1H-benzo[d]imidazol-2-yl)-2,5-dimethyl-1H-pyrrole-3-carboxamide)

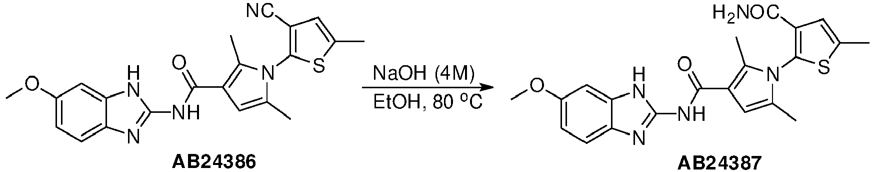

Compound **AB24386** (1-(3-cyano-5-methylthiophen-2-yl)-N-(6-methoxy-1H-benzo[d]imidazol-2-yl)-2,5-dimethyl-1H-pyrrole-3-carboxamide) (15 mg, 0.037 mmol, 1.0 eq) was dissolved in ethanol (4 mL), and sodium hydroxide (0.5 mL, 4 M in H₂O) was added thereto. The mixture was stirred at 80°C for 2 hours. After the reaction mixture was cooled, the mixture was diluted with water (10 mL) and the pH of the resulting mixture was then adjusted to 6 to 7 with 3 N hydrochloric acid. The mixture was extracted three times with ethyl acetate (20 mL × 3). The organic layers were dried over anhydrous sodium sulfate, filtered and concentrated. The residue was purified by preparative high-pressure liquid chromatography (acetonitrile/water/0.1% formic acid) so as to obtain Compound **AB24387** (1-(3-carbamoyl-5-methylthiophen-2-yl)-N-(6-methoxy-1H-benzo[d]imidazol-2-yl)-2,5- dimethyl-1H-pyrrole-3-carboxamide) as a white solid (6 mg, yield: 38.4%). MS (ESI) m/z: 424 [M+H⁺]. ¹H NMR (400 MHz, DMSO-*d*₆): *δ* 11.98 (s, 1H), 11.02 (s, 1H), 7.27 (s, 2H), 7.23 (s, 1H), 6.95 (s, 1H), 6.76 (s, 1H), 6.66 (d, *J* = 8.4 Hz, 1H), 3.71 (s, 3H), 3.31 (s, 3H), 2.31 (s, 3H), 1.94 (s, 3H).

**Example 5:** Synthesis of Compound **AB29505** (1-(2,4-dimethylthiazol-5-yl)-N-(6-methoxy-1H-benzo[d]imidazol-2-yl)-2,5-dimethyl-1H-pyrrole-3-carboxamide)

### (1) Synthetic method of Compound 2 (tert-butyl (2,4-dimethylthiazol-5-yl)carbamate)

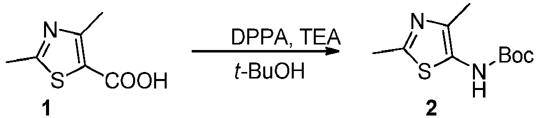

Compound **1** (2,4-dimethyl-1,3-thiazole-5-carboxylic acid) (2 g, 12.72 mmol, 1.0 eq) was dissolved in tert-butanol (40 mL). Diphenylphosphoryl azide DPPA (4.5 g, 16.53 mmol, 1.3 eq) and triethylamine (TEA) (3.2 g, 31.8 mmol, 2.5 eq) were added thereto. The mixture was stirred at 85°C for 3 hours under the protection of nitrogen gas, and then the resulting mixture was concentrated. The residue was dissolved in water (80 mL), and the resulting mixture was then extracted three times with ethyl acetate (80 mL × 3). Afterwards, the organic layers were combined, dried over anhydrous Na₂SO₄, filtered and then concentrated. The residue was purified by flash column chromatography (dichloromethane/methanol = 200/1 to 150/1) so as to obtain Compound **2** (tert-butyl (2,4-dimethylthiazol-5-yl)carbamate) as a yellow solid (2.7 g, yield: 93.1%). MS (ESI) m/z: 229.0 [M+H]⁺. TLC: dichloromethane/methanol (20:1); R_{f} (*Compound 1*) = 0.2; R_{f} (*Compound 2*) = 0.7.

### (2) Preparation method of Compound 3 (2,4-dimethylthiazole-5-amine hydrochloride)

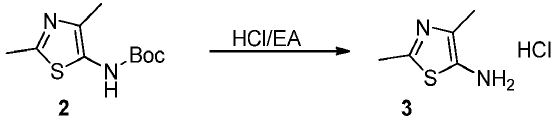

Compound **2** (tert-butyl (2,4-dimethylthiazol-5-yl)carbamate) (1.7 g, 7.44 mmol, 1.0 eq) was dissolved in a mixed solution of ethyl acetate/hydrochloric acid HCl/EA (20 mL, 4 M), and the resulting mixture was stirred at room temperature for 3 hours. Afterwards, the solid was filtered, washed twice with diethyl ether (5 mL × 2), and then concentrated, so as to obtain Compound **3** (2,4-dimethylthiazole-5-amine hydrochloride) as a yellow solid (1.1 g, yield: 91.6%). MS (ESI) m/z: 129.0 [M+H]⁺. TLC: petroleum ether/ethyl acetate (1:1); R_{f} *(Compound 2*) = 0.5; R_{f} (*Compound 3*) = 0.2.

### (3) Preparation method of Compound 5 (methyl 1-(2,4-dimethylthiazol-5-yl)- 2,5-dimethyl-1H-pyrrole-3-carboxylate)

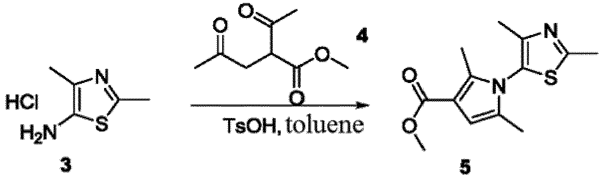

Compound **3** (2,4-dimethylthiazole-5-amine hydrochloride) (1.1 g, 6.68 mmol, 1.0 eq) was dissolved in toluene (20 mL). Compound **4** (methyl 2-acetyl-4-levulinate) (1.7 g, 10.02 mmol, 1.5 eq), 3A molecular sieve (2 g) and p-toluenesulfonic acid (459 mg, 2.67 mmol, 0.4 eq) were added thereto. The mixture was stirred at 85°C for 4 hours, and then the solid was filtered and concentrated. The residue was purified by flash column chromatography (petroleum ether/ethyl acetate = 30/1) so as to obtain Compound **5** (methyl 1-(2,4-dimethylthiazol-5-yl)-2,5-dimethyl-1H-pyrrole-3-carboxylate) as a yellow solid (1.6 g, yield: 90.9%). MS (ESI) m/z: 265 [M+H]⁺. TLC: petroleum ether/ethyl acetate (5:1); R_{f} *(Compound 3*) = 0.1; R_{f} *(Compound 5*) = 0.7.

### (4) Preparation method of Compound 6 (1-(2,4-dimethylthiazol-5-yl)-2,5-dimethyl-1H-pyrrole-3-carboxylic acid)

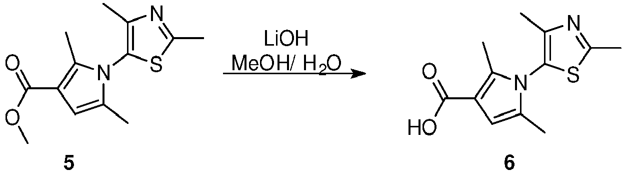

Compound **5** (1.6 g, 6.05 mmol, 1 eq) was dissolved in a solution of methanol/water MeOH/H₂O (10 mL/10 mL), and then lithium hydroxide LiOH (579 mg, 24.2 mmol, 4 eq) was added thereto. The mixture was stirred at 50°C for 1 hour. After the completion of the reaction, the resulting mixture was concentrated and diluted with water (50 mL), and the pH of the mixture was then adjusted to 5 with 3 N hydrochloric acid. The solid was filtered and concentrated, so as to obtain Compound **6** (1-(2,4-dimethylthiazol-5-yl)-2,5-dimethyl- 1H-pyrrole-3-carboxylic acid) as a yellow solid (950 mg, yield: 62.7%). MS (ESI) m/z: 251 [M+H]⁺. TLC: petroleum ether/ethyl acetate (5:1); R_{f} *(Compound 5*) = 0.7; R_{f} (*Compound 6*) = 0.2.

### (5) Preparation method of Compound AB29505 (1-(2,4-dimethylthiazol-5-yl)-N-(6-methoxy-1H-benzo[d]imidazol-2-yl)-2,5-dimethyl-1H-pyrrole-3-carboxamide)

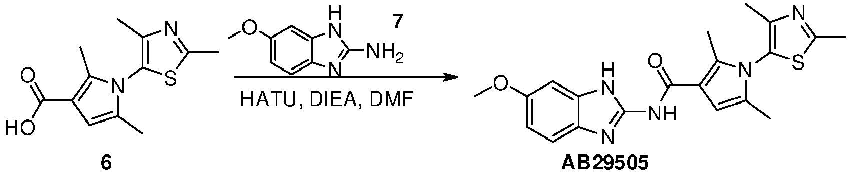

Compound **6** (1-(2,4-dimethylthiazol-5-yl)-2,5-dimethyl-1H-pyrrole-3-carboxylic acid) (100 mg, 0.399 mmol, 1.0 eq) was dissolved in dimethylformamide (5 mL). Thereafter, Compound **7** (6-methoxy-1H-benzo[d]imidazole-2-amine) (65 mg, 0.399 mmol, 1.0 eq), N,N-diisopropylethylamine DIEA (206 mg, 1.596 mmol, 4.0 eq), and O-(7-azabenzotriazol-1-yl)-N,N,N',N'-tetramethyluronium HATU (167 mg, 0.439 mmol, 1.1 eq) were added thereto. The mixture was stirred at room temperature for 16 hours. After the completion of the reaction, the resulting mixture was diluted with ethyl acetate (30 mL) and washed three times with saturated aqueous sodium chloride solution (20 mL × 3). The organic layers were dried, filtered and concentrated. The residue was purified by preparative high-pressure liquid chromatography so as to obtain Compound **AB29505** (1-(2,4-dimethylthiazol-5-yl)-N-(6-methoxy-1H-benzo[d]imidazol-2-yl)-2,5-dimethyl-1H- pyrrole-3-carboxamide) as a yellow solid (10.8 mg, yield: 6.8 %). MS (ESI) m/z: 395 [M+H]⁺. ¹H NMR (400 MHz, DMSO-*d*₆) δ 7.30 (d, J = 8.8 Hz, 1 H), 6.98 (s, 1 H), 6.83 (s, 1 H), 6.70- 6.68 (m, 1 H), 3.73 (s, 3 H), 2.64 (s, 3 H), 2.31 (s, 3 H), 2.01 (s, 3 H), 1.96 (s, 3 H).

### Example 6: Synthesis of Compound AB29510 (1-(3-cyano-4,5-dimethylfuran-2-yl)-N-(6-methoxy-1H-benzo[d]imidazol-2-yl)-2,5-dimethyl-1H-pyrrole-3-carboxamide)

### (1) Preparation method of Compound 2 (1-(3-cyano-4,5-dimethylfuran-2-yl)- 2,5-dimethyl-1H-pyrrole-3-carboxylic acid)

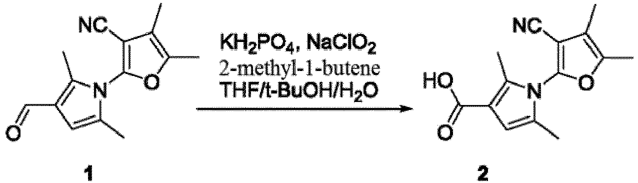

Compound **1** (2-(3-formyl-2,5-dimethyl-1H-pyrrol-1-yl)-4,5-dimethylfuran-3-carbonitrile) (1.2 g, 4.9 mmol, 1.0 eq) was dissolved in a mixed solution of tetrahydrofuran, tert-butanol and water THF/t-BuOH/ H₂O (1/1/1, 30 mL). Potassium dihydrogen phosphate KH₂PO₄ (2.9 g, 24.7 mmol, 5.0 eq), 2-methyl-1-butene (3.0 g, 49.5 mmol, 10.0 eq) and sodium chlorite (2.4 g, 29.7 mmol, 6.0 eq) were added thereto at 0°C. The mixture was stirred at room temperature for 16 hours. After the completion of the reaction, the mixture was concentrated and then purified by preparative high-pressure liquid chromatography, so as to obtain Compound **6** (1-(3-cyano-4,5-dimethylfuran-2-yl)-2,5-dimethyl-1H-pyrrole-3- carboxylic acid) as a white solid (1.0 g, yield: 78.1 %). MS (ESI) m/z: 259 [M+H⁺].

### (2) Preparation method of Compound AB29510 (1-(3-cyano-4,5-dimethylfuran-2-yl)-N-(6-methoxy-1H-benzo[d]imidazol-2-yl)-2,5-dimethyl-1H-pyrrole-3-carboxamide)

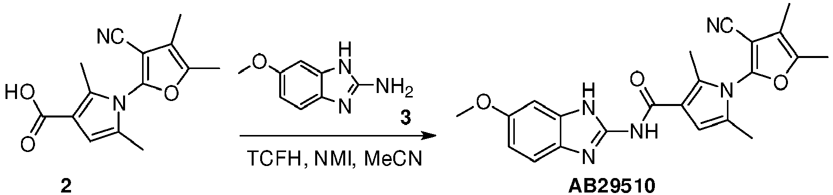

Compound **2** (1-(3-cyano-4,5-dimethylfuran-2-yl)-2,5-dimethyl-1H-pyrrole-3-carboxylic acid) (60 mg, 0.23 mmol, 1.0 eq) was dissolved in acetonitrile (2.0 mL). Compound **3** (6-methoxy-1H-benzo[d]imidazole-2-amine hydrochloride) (50 mg, 0.30 mmol, 1.3 eq), N-methylimidazole NMI (77mg, 0.94 mmol, 4.0 eq) and N,N,N',N'-tetramethylchloroformamidinium hexafluorophosphate TCFH (79 mg, 0.28 mmol, 1.2 eq) were added thereto. The mixture was stirred at room temperature for 16 hours. After the completion of the reaction, the mixture was concentrated and then purified by preparative high-pressure liquid chromatography, so as to obtain Compound **AB29510** (1-(3-cyano-4,5-dimethylfuran-2-yl)-N-(6-methoxy-1H-benzo[d]imidazol-2-yl)-2,5-dimethyl- 1H-pyrrole-3-carboxamide) as a yellow solid (2.4 mg, yield: 78.1%). MS (ESI) m/z: 404.30 [M+H⁺]. ¹H NMR (400 MHz, DMSO-*d*₆) 7.49 (d, *J* = 8.8 Hz, 1H), 7.11 (s, 1H), 6.91 (d, *J* = 8.8 Hz, 1H), 6.74 (s, 1H), 3.77 (s, 3H), 2.41 (s, 3H), 2.29 (s, 3H), 2.10 (s, 3H), 2.07 (s, 3H).

### Example 7: Synthesis of Compound AB29513 (N-(6-methoxy-1H-benzo[d]imidazol-2-yl)-2,5-dimethyl-1-((1-methyl-1H-pyrazol-4-yl)methyl)-1H-pyrrole-3-carboxamide)

### (1) Synthetic method of Compound 3 (methyl 2,5-dimethyl-1-(((1-methyl-1H-pyrazol-4-yl)methyl)-1H-pyrrole-3-carboxylate)

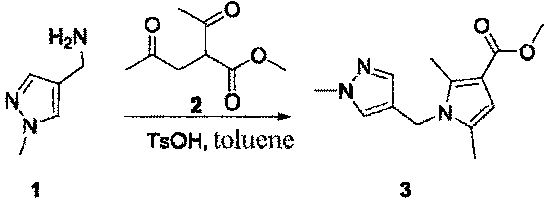

Compound **1** (4-(aminomethyl)-1-methylpyrazole) (500 mg, 4.5 mmol, 1.0 eq) was dissolved in toluene (20 mL). Compound **2** (methyl 2-acetyl-4-levulinate) (1.16 g, 6.75 mmol, 1.5 eq), 3A molecular sieve (1 g) and p-toluenesulfonic acid (310 mg, 1.8 mmol, 0.4 eq) were added thereto. The mixture was stirred at 100°C for 8 hours. The solid was filtered and concentrated. The residue was purified by flash column chromatography (petroleum ether/ethyl acetate = 10/1 to 5/1) so as to obtain Compound **3** (methyl 2,5-dimethyl-1-(((1-methyl-1H-pyrazol-4-yl)methyl)-1H-pyrrole-3-carboxylate) as a yellow solid (650 mg, yield: 58.4 %). MS (ESI) m/z: 248 [M+H]⁺. TLC: petroleum ether/ethyl acetate (2:1); R_{f} (*Compound 1*) = 0.1; R_{f} (*Compound 3*) = 0.6.

### (2) Preparation method of Compound 4 (2,5-dimethyl-1-(((1-methyl-1H-pyrazol-4-yl)methyl)-1H-pyrrole-3-carboxylic acid)

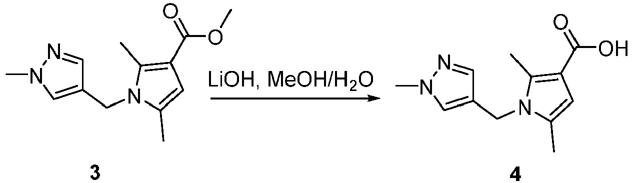

Compound **3** (methyl 2,5-dimethyl-1-(((1-methyl-1H-pyrazol-4-yl)methyl)- 1H-pyrrole- 3-carboxylate) (300 mg, 1.21 mmol, 1 eq) was dissolved in a solution of methanol/water (10 mL/10 mL), and lithium hydroxide (43 mg, 1.81 mmol, 1.5 eq) was then added thereto. The mixture was heated to 85°C and then stirred for 3 hours. The resulting mixture was concentrated and diluted with water (20 mL), and the pH of the mixture was then adjusted to 5 with 3 N hydrochloric acid. The solid was filtered and concentrated, so as to obtain Compound **4** (2,5-dimethyl-1-(((1-methyl-1H-pyrazol-4-yl)methyl)-1H-pyrrole-3- carboxylic acid) as a yellow solid (110 mg, yield: 39%). MS (ESI) m/z: 234 [M+H]⁺. TLC: dichloromethane/methanol (20:1); R_{f} *(Compound* 3) = 0.7; R_{f} (*Compound 4*) = 0.3.

### (3) Preparation method of Compound AB29513 (N-(6-methoxy-1H-benzo[d]imidazol-2-yl)-2,5-dimethyl-1-((1-methyl-1H-pyrazol-4-yl)methyl)-1H-pyrrole-3-carboxamide)

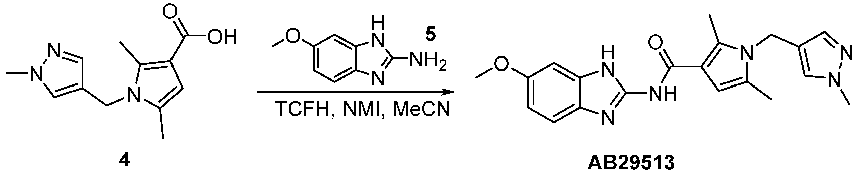

Compound **4** (2,5-dimethyl-1-(((1-methyl-1H-pyrazol-4-yl)methyl)-1H-pyrrole-3-carboxylic acid) (110 mg, 0.47 mmol, 1.0 eq) was dissolved in acetonitrile (5 mL). Compound **5** (6-methoxy-1H-benzo[d]imidazole-2-amine) (100 mg, 0.61 mmol, 1.0 eq), N-methylimidazole NMI (154 mg, 1.88 mmol, 4.0 eq) and tetramethylchloroformamidinium hexafluorophosphate TCFH (158 mg, 0.56 mmol, 1.2 eq) were added thereto. The mixture was stirred at room temperature for 16 hours. After the completion of the reaction, the mixture was diluted with ethyl acetate (30 mL) and washed three times with saturated aqueous sodium chloride solution (20 mL × 3). The organic layers were dried, filtered and concentrated. The residue was purified by preparative high-pressure liquid chromatography so as to obtain Compound **AB29513** (N-(6-methoxy-1H-benzo[d]imidazol-2-yl)-2,5-dimethyl-1- ((1-methyl-1H-pyrazol-4-yl)methyl)-1H-pyrrole-3-carboxamide) as a yellow solid (35 mg, yield: 19.6%). MS (ESI) m/z: 379 [M+H]⁺. ¹H NMR (400 MHz, DMSO-*d*₆) δ 7.55 (s, 1 H), 7.49 (d, *J* = 8.8 Hz, 1 H), 7.25 (s, 1 H), 7.13 (s, 1 H), 6.93 (d, *J* = 8.8 Hz, 1 H), 6.52 (s, 1 H), 4.90 (s, 2 H), 3.76 (d, *J* = 10.8 Hz, 6 H), 2.59 (s, 3 H), 2.23 (s, 3 H).

### Example 8: Synthesis of Compound AB29522

### (1) Synthesis of Compound 5

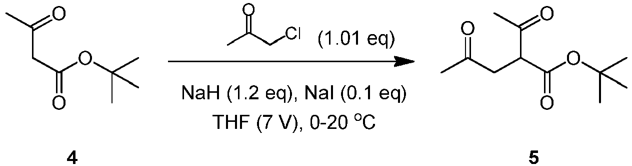

Tetrahydrofuran (25.0 mL) was added to Compound **4** (5.00 g, 31.6 mmol, 5.24 mL, 1.00 eq), and the reaction solution was cooled to 0°C, and sodium hydride (1.52 g, 37.9 mmol, 632 µl, 60% purity, 1.20 eq) was added thereto at 0°C. The reaction solution was reacted at 0°C for 30 minutes. Chloroacetone (2.95 g, 31.9 mmol, 1.01 eq) in tetrahydrofuran (10.0 mL) was slowly added dropwise into the above-mentioned reaction solution at 0°C. After the completion of the addition, sodium iodide (473 mg, 3.16 mmol, 0.100 eq) was added thereto, and the reaction solution was stirred at 20°C for 16 hours. TLC (petroleum ether: ethyl acetate =3:1, R_{f} = 0.25) showed that some raw materials remained, and a new spot with higher polarity was generated. After the completion of the reaction, the reaction solution was slowly poured into saturated aqueous ammonium chloride solution for quenching, followed by extraction with ethyl acetate for three times (50.0 mL × 3), and the organic phases were combined and washed once with saturated saline solution (30.0 mL). The separated organic phases were dried over anhydrous sodium sulfate and then concentrated. The crude product was separated and purified by column chromatography (petroleum ether: ethyl acetate =10:1 to 1:1), so as to obtain Compound 5 (4.00 g, 16.8 mmol, yield: 53.16%, purity: 90%) as a light yellow oil.

### (2) Synthesis of Compound 2

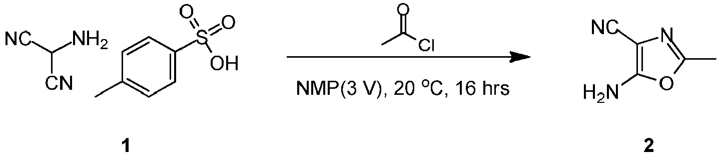

1-Methyl-2-pyrrolidone (10 mL) was added to Compound 1 (3.00 g, 11.8 mmol, 1.00 eq), and acetyl chloride (1.12 g, 14.2 mmol, 1.01 mL, 1.20 eq) was added to the reaction solution, which was stirred at 20°C for 16 hours. The generation of a spot showing strong ultraviolet absorption was detected by TLC (petroleum ether: ethyl acetate = 1: 1). LCMS showed that the peak of the target product was detected. Water (30.0 mL) was added to the reaction solution, and the pH of the resulting mixture was then adjusted to 7 to 8 with saturated aqueous sodium bicarbonate solution. The mixture was extracted twice with ethyl acetate (50.0 mL × 2), and the organic phases were combined and washed once with saturated saline solution (20.0 mL). The separated organic phases were dried over anhydrous sodium sulfate and then concentrated, so as to obtain Compound **2** (1.66 g, crude) as a yellow solid (LCMS: Ms: M + H⁺ = 124.5), which was directly used in the next reaction.

### (3) Synthesis of Compound 3

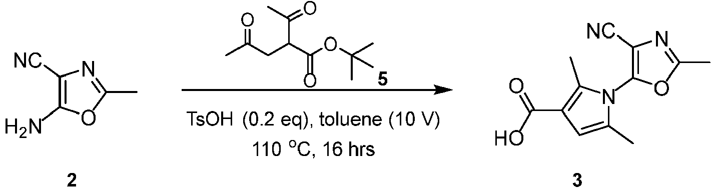

Toluene (5.00 mL) was added to Compound **2** (500 mg, 4.06 mmol, 1.00 eq), and Compound **5** (870 mg, 4.06 mmol, 1.00 eq) and anhydrous p-toluenesulfonic acid (140 mg, 812 µmol, 0.200 eq) were added into the reaction solution, which was stirred at 110°C for 16 hours. LCMS showed that the raw materials were consumed, and the main peak of the target product was detected. The reaction solution was directly added with water (30.0 mL) and then extracted twice with ethyl acetate (50.0 mL × 2), and the organic phases were combined and washed once with saturated saline solution (20.0 mL). The separated organic phases were dried over anhydrous sodium sulfate and then concentrated. Ethyl acetate (20.0 mL) was added to the crude product. The mixture was stirred for 20 minutes and then filtered, and 300 mg of filter cake was collected. The filtrate was separated and purified by column chromatography (petroleum ether: ethyl acetate = 10: 1 to 1: 1) so as to obtain 140 mg of Compound **3** (440 mg, 1.70 mmol, yield: 41.97%, purity: 95%) as a gray solid. ¹H NMR (400 MHz, CDCl₃) δ ppm 6.47 (s, 1 H), 2.58 (s, 3 H), 2.47 (s, 3 H), 2.15 (s, 3 H).

### (4) Synthesis of Compound AB29522

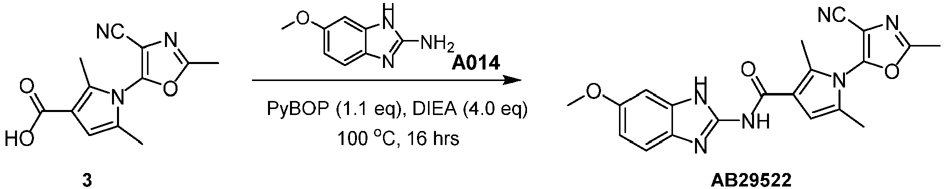

N,N-dimethylformamide (1.00 mL) was added to Compound **3** (50.0 mg, 204 µmol, 1.00 eq), and Compound **A014** (33.3 mg, 204 µmol, 1.00 eq), N,N-diisopropylethylamine (105 mg, 815 µmol, 142 µL, 4.00 eq), benzotriazol-1-yl-oxytripyrrolidinophosphonium hexafluorophosphate (117 mg, 224 µmol, 1.10 eq) were added to the reaction solution, which was stirred at 100°C for 16 hours. LCMS showed that the raw materials were consumed, and the peak of the target product was detected. The reaction solution was directly concentrated. The crude product was separated and purified by high performance liquid chromatography (column: Welch Xtimate C18 150 × 25mm × 5µm; mobile phase: [water (ammonia hydroxide v/v)-ACN]; B%: 27%-57%, 2 minutes), so as to obtain Compound **AB29522** (20.0 mg, 48.05 µmol, yield: 23.57%, purity: 93.8%) as a brown solid. ¹H NMR (400 MHz, DMSO-d₆) δ ppm 11.83 - 12.34 (m, 1 H), 10.96 - 11.64 (m, 1 H), 7.33 (d, *J* = 8.63 Hz, 1 H), 7.01 (br s, 1 H), 6.92 (s, 1 H), 6.73 (dd, *J* = 8.63, 2.38 Hz, 1 H), 3.76 (s, 3 H), 2.57 (s, 3 H), 2.50 (s, 3 H), 2.17 (s, 3 H). LCMS: Ms: M +H⁺ = 391.

### Example 9 Synthesis of Compound AB29553

### (1) Synthesis of Compound 2

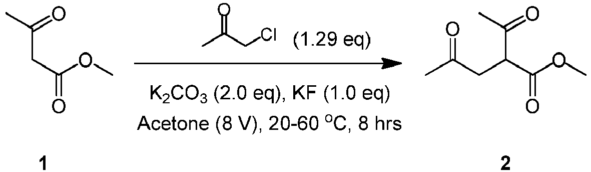

Acetone (140 mL) was added to Compound **1** (25.0 g, 215 mmol, 23.1 mL, 1.00 eq), potassium carbonate (59.5 g, 430 mmol, 2.00 eq) and potassium fluoride (12.5 g, 215 mmol, 1.00 eq) were added to the reaction solution, and the reaction mixture was stirred at 60°C for 1 hour. Then, the reaction solution was cooled to 20°C, and a solution of chloroacetone (25.7 g, 278 mmol, 1.29 eq) dissolved in acetone (35.0 mL) was slowly added dropwise to the reaction mixture. After the completion of the addition, the reaction mixture was heated to 60°C and stirred for 7 hours. TLC (petroleum ether: ethyl acetate = 5: 1, Rf = 0.38) showed that Compound 1 had been reacted completely, and that a new spot with higher polarity was detected. The reaction solution was cooled to room temperature and filtered. The collected filtrate was slowly poured into water (500 mL), and extracted with ethyl acetate (500 mL × 3), and the combined organic phases were washed with saturated saline solution (200 mL). The separated organic phases were dried over anhydrous sodium sulfate and then filtered and concentrated. The crude product was separated and purified by column chromatography (silica, petroleum ether/ethyl acetate = 20/1 to 1/1), so as to obtain Compound **2** (21.0 g, 122 mmol, yield: 56.6%) as a light yellow liquid. ¹H NMR (400 MHz, CDCl₃) δ ppm 4.03 (dd, *J* = 8.19, 5.69 Hz, 1 H), 3.74 (s, 3 H), 3.09 - 3.22 (m, 1 H), 2.89 - 3.02 (m, 1 H), 2.36 (s, 3 H), 2.19 (s, 3 H).

### (2) Synthesis of Compound 4

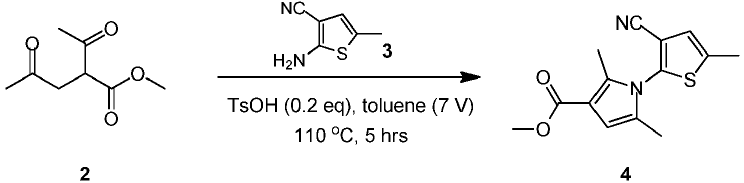

Toluene (70.0 mL) was added to Compound **2** (10.0 g, 58.0 mmol, 1.00 eq), and Compound **3** (8.83 g, 63.9 mmol, 1.10 *eq)* and anhydrous p-toluenesulfonic acid (2.00 g, 11.6 mmol, 0.2 eq) were added to the reaction solution, which was stirred at 110°C for five hours. TLC (petroleum ether: ethyl acetate =10: 1, Rf = 0.43) showed that Compound **2** had been reacted completely, and that a new spot with lower polarity was generated. The reaction solution was directly concentrated, and water (100 mL) was added to the concentrated residue, which was extracted three times with ethyl acetate (100 mL × 3). The combined organic phases were washed once with saturated saline solution (50.0 mL), and the separated organic phases were dried over anhydrous sodium sulfate, and then filtered and concentrated. The crude product was separated and purified by column chromatography (silica, petroleum ether: ethyl acetate = 20: 1 to 1: 1), so as to obtain Compound **4** (11.2 g, 39.5 mmol, yield: 67.9%) as a yellow solid.

### (3) Synthesis of Compound 5

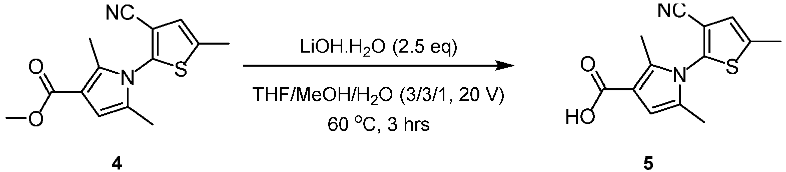

Compound **4** (6.00 g, 21.9 mmol, 1.00 eq) was dissolved in tetrahydrofuran (20.0 mL) and methanol (20 mL), and a solution of lithium hydroxide monohydrate (2.29 g, 54.7 mmol, 2.50 eq) dissolved in water (6.00 mL) was added to the above reaction solution. The reaction mixture was stirred at 60°C for 3 hours. LCMS showed that about 25% of Compound **4** remained, and 50% of the target product was detected. The reaction solution was concentrated under a water pump to remove the solvent. After concentration, 2N hydrochloric acid solution was slowly added to the residue until the pH was 5 to 6, then the mixture was extracted twice with ethyl acetate (80.0 mL × 2), and the organic phases were washed once with saturated saline solution (50.0 mL). The separated organic phases were dried over anhydrous sodium sulfate and then concentrated. The crude product was separated and purified by column chromatography (silica, petroleum ether/ethyl acetate = 10/1 to 1/1), so as to obtain Compound **5** (2.10 g, 8.07 mmol, yield: 36.9%) as a yellow solid (LCMS: Ms: M +H⁺ = 261.3).

### (4) Synthesis of Compound AB29553

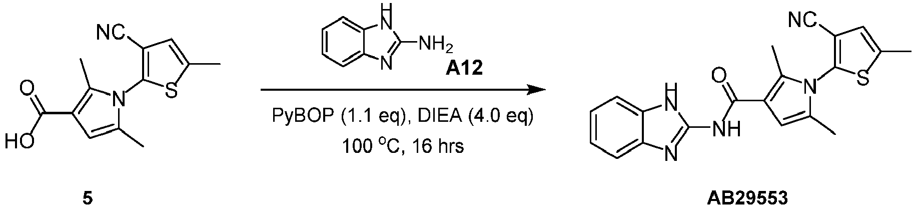

N,N-dimethylformamide (1 mL) was added to Compound **5** (50.0 mg, 192 µmol, 1.00 eq), and diisopropylethylamine (99.3 mg, 768 µmol, 134 µL, 4.00 eq) and Compound **A12** (25.6 mg, 192 µmol, 1.00 eq) were added to the reaction solution, followed by the addition of benzotriazol-1-yl-oxytripyrrolidinophosphonium hexafluorophosphate (110 mg, 211µmol, 1.10 eq). The reaction mixture was stirred at 100°C for 16 hours. LCMS showed that the raw materials were reacted completely, and a peak of the target product was detected. The reaction solution was directly concentrated. The crude product was separated and purified by high performance liquid chromatography (column: Welch Ultimate C18 150 × 25mm × 5µm; mobile phase: [water (TFA) - ACN]; B%: 26% to 56%, 10 minutes), so as to obtain **Compound AB29553** (15.0 mg, 30.3 µmol, yield: 15.8%, purity: 98.9%, trifluoroacetate) as a white solid. ¹H NMR (400 MHz, DMSO-*d*₆) δ ppm 7.61 (dd, *J* = 5.88, 3.13 Hz, 2 H), 7.37 (d, *J =* 1.13 Hz, 1 H), 7.29 (br dd, *J =* 5.44, 2.81 Hz, 2 H), 6.84 (s, 1 H), 2.56 (br d, *J* = 1.00 Hz, 3 H), 2.44 (s, 3 H), 2.11 (s, 3 H). LCMS: Ms: M +H⁺ = 376.

### Example 10 Synthesis of Compound AB29556

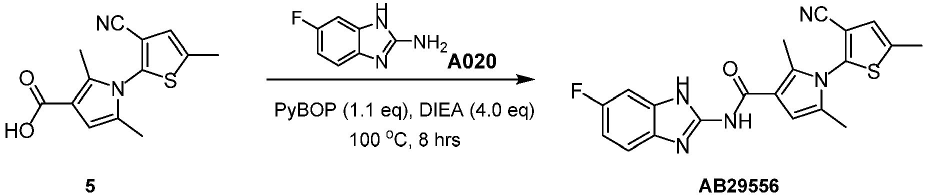

N,N-dimethylformamide (1.00 mL) was added to Compound **5** (60.0 mg, 230 µmol, 1.00 eq) obtained in Example 16, and N,N-diisopropylethylamine (119 mg, 922 µmol, 160 µL, 4.00 eq) was added to the reaction solution, followed by the addition of Compound **A020** (34.8 mg, 230 µmol, 1.00 eq), benzotriazol-1-yl-oxytripyrrolidinophosphonium hexafluorophosphate (132 mg, 253 µmol, 1.10 eq). The reaction solution was stirred at 100°C for 8 hours. LCMS showed that the raw materials were reacted completely, and a peak of the target product was detected. The reaction solution was concentrated under an oil pump. The crude product was separated and purified by high performance liquid chromatography (column: Welch Ultimate C18 150 × 25mm × 5µm; mobile phase: [water (TFA) - ACN]; B%: 30% to 60%, 15 minutes), followed by secondary purification (column: waters xbridge 150 × 25mm 10µm; mobile phase: [water (NH₄HCO₃) - ACN]; B%: 38% to 68%, 8 minutes), so as to obtain **Compound AB29556** (6.03 mg, 15.2 µmol, yield: 6.58%, purity: 99%) as a white solid. ¹H NMR (400 MHz, DMSO-d₆) δ ppm 12.29 (br s, 1 H), 11.33 (td, J = 6.13, 1.63 Hz, 1 H), 7.38 - 7.51 (m, 1 H), 7.35 (d, *J =* 1.13 Hz, 1 H), 7.14 - 7.31 (m, 1 H), 6.83 - 7.06 (m, 2 H), 2.55 (br d, J = 0.88 Hz, 3 H), 2.41 (s, 3 H), 2.06 (s, 3 H). LCMS: Ms: M +H⁺ = 394.

### Example 11 Synthesis of Compound AB29557

### (1) Synthesis of Compound 2

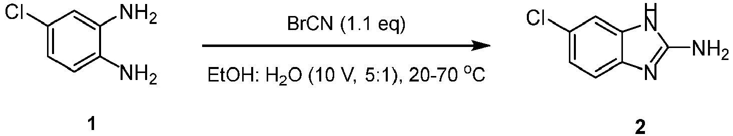

Water (1.00 mL) and ethanol (5.00 mL) were added to Compound **1** (500 mg, 3.51 mmol, 1.00 eq), and cyanogen bromide (446 mg, 4.21 mmol, 309 µL, 1.20 eq) was added to the reaction solution at 20°C, and the reaction solution was stirred at 70°C for 2 hours. LCMS showed that the raw materials were consumed, and a main peak of the target product was detected. After the completion of the reaction, the reaction solution was directly concentrated, and ethyl acetate (30.0 mL) was added to the crude product, which was stirred for 30 minutes and then filtered. The filter cake was washed twice with ethyl acetate (5.00 mL × 2), and the filter cake was collected, so as to obtain Compound **2** (700 mg, 2.68 mmol, yield: 76.3%, purity: 95%, hydrobromide) as a black solid. ¹H NMR (400 MHz, MeOD) δ ppm 7.39 (d, *J* = 1.38 Hz, 1 H), 7.32 - 7.36 (m, 1 H), 7.25 - 7.30 (m, 1 H). LCMS: Ms: M +H⁺ = 168.

### (2) Synthesis of Compound AB29557

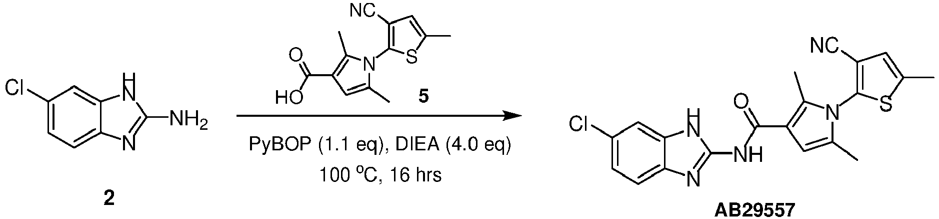

N,N-dimethylformamide (1.00 mL) was added to **Compound 2** (50.0 mg, 192 µmol, 1.00 eq), followed by addition of **Compound 5** (35.4 mg, 211 µmol, 1.10 eq) and N,N-diisopropylethylamine (99.3 mg, 768 µmol, 134 µL, 4.00 eq) to the reaction solution. Then, benzotriazol-1-yl-oxytripyrrolidinophosphonium hexafluorophosphate (110 mg, 211 µmol, 1.10 eq) was added to the reaction solution, and the reaction solution was stirred at 100°C for 16 hours. LCMS showed that the raw materials were consumed, and a peak of the target product was detected. The reaction solution was concentrated under an oil pump. The crude product was separated and purified by high performance liquid chromatography (column: Welch Xtimate C18 150 × 25mm × 5µm; mobile phase: [water (NH₄HCO₃) - ACN]; B%: 43% to 73%, 2 minutes), so as to obtain **Compound AB29557** (0.022 g, 50.9 µmol, yield: 26.5%, purity: 94.9%) as a yellow solid. ¹H NMR (400 MHz, DMSO-*d*₆) δ ppm 12.31 - 12.45 (m, 1 H), 11.38 - 11.45 (m, 1 H), 7.39 - 7.54 (m, 2 H), 7.36 (d, *J =* 1.13 Hz, 1 H), 7.08 - 7.16 (m, 1 H), 6.95 (s, 1 H), 2.55 (br s, 3 H), 2.42 (s, 3 H), 2.07 (s, 3 H). LCMS: Ms: M +H⁺ = 410.

### Example 12: Synthesis of Compound AB29558

### (1) Synthesis of Compound 2

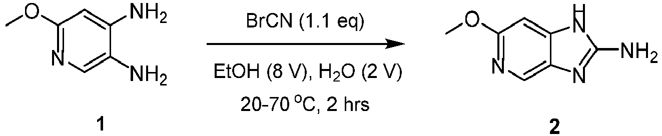

Compound **1** (350 mg, 2.52 mmol, 1.00 eq) was dissolved in ethanol (2.00 mL) and water (0.40 mL), and hydrogen bromide (0.26 g, 2.45 mmol, 180 µL, 1.10 eq) was slowly added to the above mixture at 20°C. The reaction solution was stirred at 70°C for 2 hours. LCMS showed that Compound **1** was consumed, and the molecular weight of the desired compound was detected. The reaction solution was concentrated under vacuum. Purification was carried out by thin layer chromatography (dichloromethane: methanol = 5: 1, 2.00 mL ammonia water) so as to obtain Compound **2** (80.0 mg, 487 µmol, yield: 19.3%) as a brown oil. LCMS: Ms: M +H⁺ = 165.

### (2) Synthesis of Compound AB29558

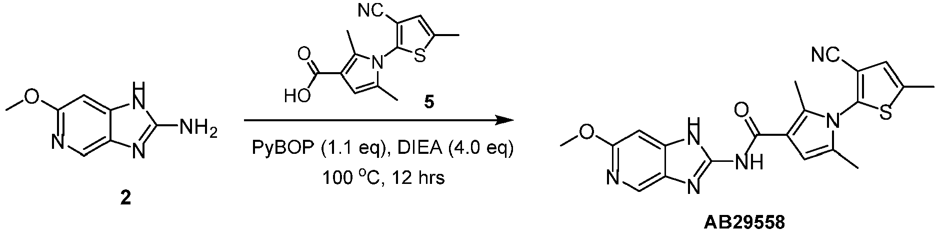

Compound **2** (69.3 mg, 266 µmol, 1.00 eq) was dissolved in N,N-dimethylformamide (2.00 mL), followed by the addition of Compound **5** (70.0 mg) and N,N- diisopropylethylamine (137 mg, 1.07 mmol, 185 µL, 4.00 eq), and benzotriazol-1-yl-oxytripyrrolidinophosphonium hexafluorophosphate (152 mg, 293 umol, 1.10 eq) was added to the reaction solution, and the reaction mixture was stirred at 100°C for 12 hours. LCMS showed that the raw materials were consumed, and the molecular weight of the desired compound was detected. The reaction solution was concentrated under vacuum. The first purification was carried out by high performance liquid chromatography (column: Welch Xtimate C18 150 × 25mm × 5µm; mobile phase: [water (NH₃H₂O) - ACN]; B%: 25% to 55%, 8 minutes). The secondary purification was carried out by high performance liquid chromatography (column: Welch Xtimate C18 150 × 25mm × 5µm; mobile phase: [water (HCl) - ACN]; B%: 16% to 46%, 8 minutes), so as to obtain Compound **AB29558** (8.68 mg, 19.4 µmol, yield: 7.28%, purity: 99%, hydrochloride) as a white solid. 1H NMR (400 MHz, DMSO-d6) δ ppm 11.68 - 11.92 (m, 1.00 H), 8.27 (br s, 1.00 H), 7.25 (d, *J =* 1.13 Hz, 1.00 H), 6.96 - 7.06 (m, 1.00 H), 6.86 (s, 1.00 H), 3.89 (s, 3.00 H), 2.44 (d, *J* = 1.00 Hz, 3.00 H), 2.31 (s, 3.00 H), 1.97 (s, 3.00 H). LCMS: Ms: M +H⁺ = 407.

### Example 13: Synthesis of Compound AB29559

### (1) Synthesis of Compound 2

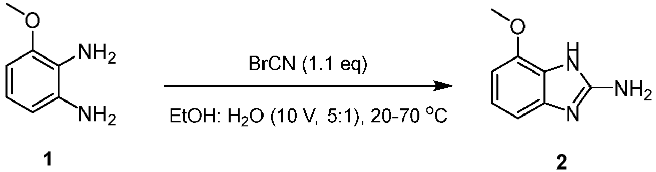

Ethanol (5.00 mL) and water (1.00 mL) were added to **Compound 1** (500 mg, 3.62 mmol, 1.00 eq), and hydrogen bromide (460 mg, 4.34 mmol, 319 µL, 1.20 eq) was added to the reaction solution at 20°C. The reaction solution was stirred at 70°C for 3 hours. LCMS showed that the raw materials were consumed, and the main peak of the target product was detected. After the completion of the reaction, the reaction solution was directly concentrated, and ethyl acetate (30 mL) was added to the crude product and stirred for half an hour, then filtered. The filter cake was washed twice with ethyl acetate (5.00 mL × 2), and the filter cake was collected so as to obtain **Compound 2** (700 mg, crude) as a brown solid (LCMS: Ms: M +H⁺ = 164.2).

### (2) Synthesis of Compound AB29559

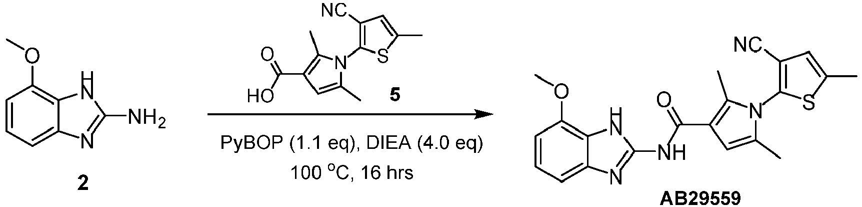

N,N-dimethylformamide (1.00 mL) was added to **Compound 2** (60.0 mg, 230 µmol, 1.00 eq), followed by addition of N,N-diisopropylethylamine (119 mg, 922 µmol, 160 µL, 4.00 eq) and **Compound 5** (37.6 mg, 230 µmol, 1.00 eq) to the reaction solution. Then, benzotriazol-1-yl-oxytripyrrolidinophosphonium hexafluorophosphate (132 mg, 253 µmol, 1.10 eq) was added to the reaction solution, and the reaction mixture was stirred at 100°C for 16 hours. LCMS showed that the raw materials were consumed, and the main peak of the target product was detected. The reaction solution was directly concentrated. The crude product was separated and purified by high performance liquid chromatography (column: Welch Ultimate C18 150 × 25mm × 5µm; mobile phase: [water (FA) - ACN]; B%: 32% to 62%, 10 minutes), so as to obtain **Compound AB29559** (20.0 mg, 43.8 µmol, yield: 19.0%, purity: 98.9%, formate) as an off-white solid. ¹H NMR (400 MHz, DMSO-*d*₆) δ ppm 12.04 - 13.00 (m, 1 H), 11.04 - 11.86 (m, 1 H), 8.14 (s, 1 H), 7.36 (d, *J =* 1.13 Hz, 1 H), 7.12 - 7.18 (m, 1 H), 7.02 - 7.09 (m, 1 H), 6.90 (s, 1 H), 6.73 (d, *J* = 8.00 Hz, 1 H), 3.93 (s, 3 H), 2.55 (d, *J* = 0.75 Hz, 3 H), 2.42 (s, 3 H), 2.08 (s, 3 H). LCMS: Ms: M +H⁺ = 406.

### Example 14: Synthesis of Compound AB29560

### (1) Synthesis of Compound 2

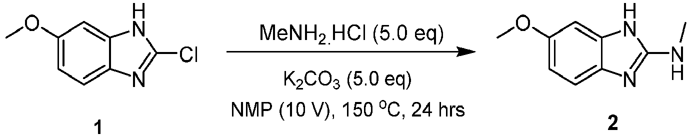

Methylamine hydrochloride (1.44 g, 21.4 mmol, 5.00 eq, HCl) was dissolved in N-methylpyrrolidone (10.0 mL), followed by addition of **Compound 1** (780 mg, 4.27 mmol, 1.00 eq) and potassium carbonate (2.95 g, 21.4 mmol, 5.00eq). The mixture was stirred at 150°C for 24 hours. LCMS showed that **Compound 1** was consumed, and the molecular weight of the desired compound was detected. Water (50.0 mL) was added into the reaction solution, then ethyl acetate (50 mL × 3) was added to perform extraction for three times. The organic phases were combined and washed once with saturated saline solution (20.0 mL), and the separated organic phases were dried over anhydrous sodium sulfate and concentrated under reduced pressure so as to obtain **Compound 2** (1.00 g, crude) as a crude product (LCMS: Ms: M +H⁺ = 178.2), which was directly used in the next step.

### (2) Synthesis of Compound AB29560

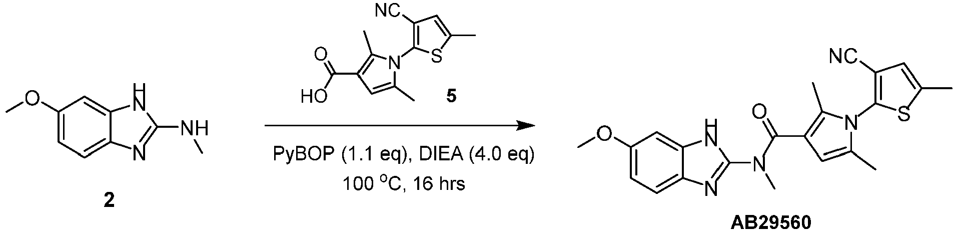

N,N-dimethylformamide (1.00 mL) was added to **Compound 5** (0.05 g, 192 µmol, 1.00 eq), and **Compound 2** (34.04 mg, 192 µmol, 1.00 eq), N,N-diisopropylethylamine (99.3 mg, 768 µmol, 134 µL, 4.00 eq), benzotriazol-1-yl-oxytripyrrolidinophosphonium hexafluorophosphate (110 mg, 211 µmol, 1.10 eq) were added to the reaction solution, which was stirred at 100°C for 16 hours. LCMS showed that the raw materials were consumed, and the main peak of the target product was detected. The reaction solution was directly concentrated. The crude product was separated and purified by high performance liquid chromatography (column: Phenomenx C18 150 × 25mm × 10µm; mobile phase: [water (NH₄HCO₃) - ACN]; B%: 36% to 66%, 10 minutes), so as to obtain **Compound AB29560** (23.0 mg, 52.9 µmol, yield: 13.8%, purity: 96.5%) as an off-white solid. ¹H NMR (400 MHz, DMSO-*d*₆) δ ppm 12.20 (br s, 1 H), 7.29 - 7.44 (m, 2 H), 6.97 - 7.11 (m, 1 H), 6.76 (dd, *J* = 8.69, 2.19 Hz, 1 H), 5.98 - 6.11 (m, 1 H), 3.77 (s, 3 H), 3.56 (br d, *J* = 11.76 Hz, 3 H), 2.54 (s, 3 H), 2.22 (s, 3 H), 1.99 (br d, *J* = 4.75 Hz, 3 H). LCMS: Ms: M +H⁺ = 420.

### Example 15: Synthesis of Compound AB29561

### (1) Synthesis of Compound 2

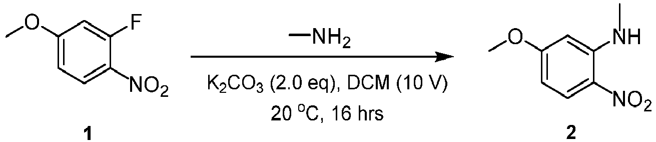

Compound **1** (5.00 g, 29.2 mmol, 1.00 eq) was dissolved in dichloromethane (50.0 mL), and methylamine (3.95 g, 58.4 mmol, 2.00 eq, hydrochloride) and potassium carbonate (6.06 g, 43.8 mmol, 1.50 eq) were added thereto. Then, the reaction mixture was stirred at 20°C for 12 hours. TLC (petroleum ether: ethyl acetate = 3: 1) showed that Compound **1** was consumed, and a new spot with higher polarity was detected. Purification was carried out by thin layer chromatography (petroleum ether: ethyl acetate = 3: 1) so as to obtain Compound **2** (3.50 g, 82.3 µmol, yield: 70.0%) as a yellow solid.

### (2) Synthesis of Compound 3

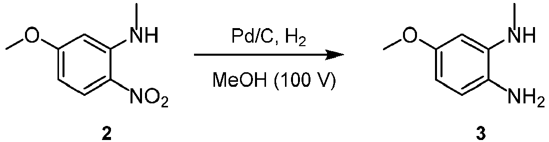

**Compound 2** (5.00 g, 27.4 mmol, 1.00 eq) was dissolved in methanol (500 mL), and Pd/C (2.00 g, 10% purity) was added thereto. The mixture was purged with hydrogen three times and stirred at 25°C and 15 Psi for 12 hours. LCMS showed that Compound **2** was consumed, and the molecular weight of the desired compound was detected. The reaction solution was filtered under vacuum, and the filtrate was collected and concentrated under reduced pressure so as to obtain **Compound 3** (3.5 g, crude product) as a brown oil (LCMS: Ms: M +H⁺ = 153.2).

### (3) Synthesis of Compound 4

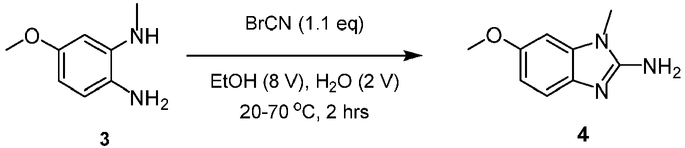

**Compound 3** (500 mg, 3.29 mmol, 1.00 eq) was dissolved in water (1.00 mL) and ethanol (5.00 mL), and then hydrogen bromide (417 mg, 3.94 mmol, 289 µL, 1.20 eq) was slowly added to the above mixture at 25°C. The reaction system was slowly stirred at 70°C for 4 hours. LCMS showed that **Compound 3** was consumed, and the molecular weight of the desired compound was detected. The reaction solution was concentrated under vacuum, and ethyl acetate (20.0 mL) was added thereto and stirred, followed by filtration under vacuum. The filter cake was collected and concentrated under reduced pressure so as to obtain **Compound 4** (340 mg, crude product) as a white solid (LCMS: Ms: M +H⁺ = 178.1).

### (4) Synthesis of Compound AB29561

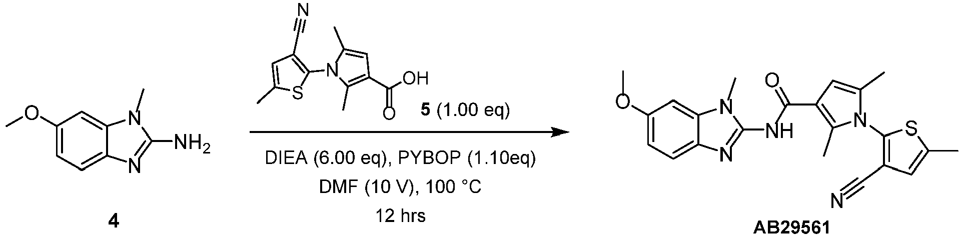

**Compound 4** (163 mg, 921 µmol, 3.00 eq) was dissolved in N,N-dimethylformamide (2.00 mL), N,N-diisopropylethylamine (238 mg, 1.84 mmol, 321 µL, 6.00 eq) was added thereto, and **Compound 5** (80.0 mg, 307 µmol, 1.00 eq) and benzotriazol-1-yl-oxytripyrrolidinophosphonium hexafluorophosphate (175 mg, 338 µmol, 1.10 eq) were added to the reaction solution, and the mixture was stirred at 100°C for 12 hours. LCMS showed that the raw materials were consumed and the molecular weight of the target product was detected. The reaction solution was concentrated under vacuum. The crude product was purified by high performance liquid chromatography (column: waters xbridge 150 × 25 mm 10 µm; mobile phase: [water (NH₄HCO₃) - ACN]; B%: 41% to 71%, 9 minutes), so as to obtain **Compound AB29561** (10.9 mg, 26.1 µmol, yield: 8.50% ) as a white solid. ¹H NMR (400 MHz, DMSO-d6) δ ppm 12.32 - 12.45 (m, 1 H), 7.27 - 7.34 (m, 2 H), 7.04 (br d, *J* = 2.00 Hz, 1 H), 6.75 (br dd, *J* = 8.76, 2.13 Hz, 1 H), 6.51 (s, 1 H), 3.79 (s, 3 H), 3.59 (s, 3 H), 2.52 (s, 6 H), 1.98 - 2.09 (m, 3 H). LCMS: Ms: M +H⁺ = 420.

### Example 16 Synthesis of Compounds AB29562 and AB29563

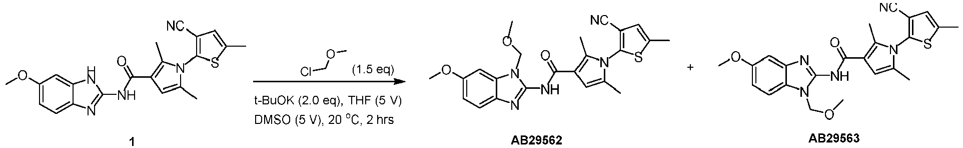

Tetrahydrofuran (0.5 mL) and dimethyl sulfoxide (0.5 mL) were added to Compound **1** (100 mg, 246 µmol, 1.00 eq), potassium tert-butoxide (55.2 mg, 493 µmol, 2.00 eq) was added to the reaction solution, and then chloromethyl methyl ether (29.8 mg, 369 µmol, 28.1 µl, 1.50 eq) was added dropwise to the reaction solution, which was then stirred at 20°C for 2 hours. LCMS showed that the raw materials were consumed, and the peak of the target product was detected. After the completion of the reaction, water (5.00 mL) was directly added to the reaction solution, which was then extracted twice with ethyl acetate (5.00 mL × 2), and the organic phases were combined, dried and concentrated. The crude product was separated and purified by high performance liquid chromatography (column: Welch Xtimate C18 150 × 25mm × 5 µm; mobile phase: [water (NH₄HCO₃) - ACN]; B%: 42% to 72%, 10 minutes), and the obtained product was separated by SFC (column: DAICEL CHIRALCEL OD (250 mm × 30 mm, 10 µm); mobile phase: [0.1% NH₃H₂O MEOH]; B%: 35% to 35%, C6.8; 68 minutes), so as to obtain **Compound AB29562** (5.49 mg, 11.66 µmol, yield: 4.73%, purity: 95.51%) as an off-white solid, and **Compound AB29563** (7.44 mg, 15.9 µmol, yield: 6.47%, purity: 96.38%) as an off-white solid. **CompoundAB29562:** ¹H NMR (400 MHz, DMSO-*d*₆) δ ppm 7.40 (br d, *J* = 8.25 Hz, 1 H), 7.26 (s, 1 H), 7.04 (br s, 1 H), 6.82 (br d, *J* = 8.25 Hz, 1 H), 6.55 (br s, 1 H), 5.57 (br s, 2 H), 3.83 (s, 3 H), 3.37 (s, 3 H), 2.56 (s, 3 H), 2.50 (br s, 3 H), 2.08 (s, 3 H). LCMS: Ms: M +H⁺ = 450. **Compound AB29563:** ¹H NMR (400 MHz, DMSO-*d*₆) δ ppm 12.41 - 12.65 (m, 1 H), 7.27 - 7.41 (m, 2 H), 7.10 (d, *J* = 2.38 Hz, 1 H), 6.82 (dd, *J* = 8.76, 2.38 Hz, 1 H), 6.54 (s, 1 H), 5.55 (s, 2 H), 3.69 - 3.84 (m, 3 H), 3.30 (s, 3 H), 2.54 (s, 3 H), 2.48 (br s, 3 H), 2.05 (s, 3 H). LCMS: Ms: M +H⁺ = 450.

### Example 17: Synthesis of Compounds AB29564 and AB29565

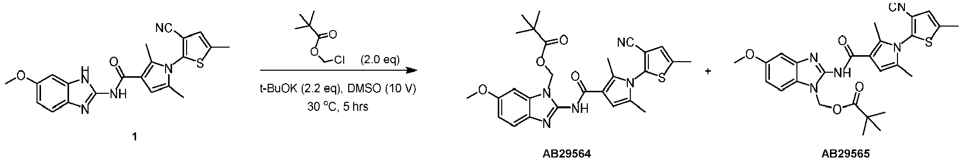

Dimethyl sulfoxide (1.00 mL) was added to Compound **1** (100 mg, 246 µmol, 1.00 eq), potassium tert-butoxide (60.9 mg, 542 µmol, 2.20 eq) was added thereto, and then chloromethyl trimethyl chloroacetate (74.3 mg, 493 µmol, 71.4 µL, 2.00 eq) was added to the reaction solution, which was stirred at 30°C for 5 hours. LCMS showed that approximately 5% of the raw materials remained, and a main peak of the target product was detected. Water (10.0 mL) was added to the reaction solution, which was then extracted twice with ethyl acetate (20.0 mL × 2). The combined organic phases were washed once with saturated saline solution (10.0 mL), and the separated organic phases were dried over anhydrous sodium sulfate and concentrated. The crude product was separated and purified by high performance liquid chromatography (column: Welch Xtimate C18 150 × 25 mm × 5 µm; mobile phase: [water (NH₄HCO₃) - ACN]; B%: 55% to 85%, 8 minutes), and the obtained product was separated by SFC (column: DAICEL CHIRALCEL OD (250 mm × 30 mm, 10 µm); mobile phase: [0.1% NH₃H₂O MeOH]; B%: 35% to 35%, C7.5; 60 minutes), so as to obtain **Compound AB29564** (5.36 mg, 9.62 µmol, yield: 3.90%, purity: 93.2%) as an off-white solid, and **CompoundAB29565** (5.39 mg, 9.81 µmol, yield 3.98%, purity 94.6%) as an off-white solid.

**Compound AB29564:** ¹H NMR (400 MHz, DMSO-*d*₆) δ ppm 7.38 (br d, *J* = 8.63 Hz, 1 H), 7.25 (s, 1 H), 7.12 (s, 1 H), 6.79 - 6.94 (m, 1 H), 6.52 (s, 1 H), 6.06 - 6.30 (m, 2 H), 3.82 (s, 3 H), 2.55 (s, 3 H), 2.49 (br s, 3 H), 2.07 (s, 3 H), 1.14 (s, 9 H). LCMS: Ms: M +H⁺ = 520.

**Compound AB29565:** ¹H NMR (400 MHz, DMSO-*d*₆) δ ppm 12.32 - 12.70 (m, 1 H), 7.39 (br d, *J =* 8.38 Hz, 1 H), 7.31 (s, 1 H), 7.08 (br s, 1 H), 6.84 (br d, *J* = 8.38 Hz, 1 H), 6.50 (br s, 1 H), 6.16 (br s, 2 H), 3.77 (s, 3 H), 2.53 (s, 3 H), 2.46 (br s, 3 H), 2.05 (s, 3 H), 1.09 (s, 9 H). LCMS: Ms: M +H⁺ = 520.

### Example 18 Synthesis of Compound AB29572

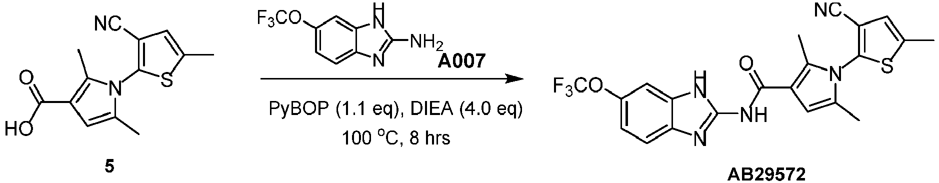

N,N-dimethylformamide (1.00 mL) was added to Compound **5** (50.0 mg, 192 µmol, 1.00 eq), and Compound **A007** (41.7 mg, 192 µmol, 1.00 eq), benzotriazol-1-yl-oxytripyrrolidinophosphonium hexafluorophosphate (110 mg, 211 µm, 1.10 eq) and N,N-diisopropylethylamine (99.3 mg, 768 µmol, 134 µL, 4.00 eq) were added to the reaction solution, which was stirred at 100°C for 8 hours. LCMS showed that the raw materials were consumed, and the peak of the target product was detected. The reaction solution was concentrated under an oil pump. The crude product was separated and purified by high performance liquid chromatography (column: Welch Xtimate C18 150 × 25 mm × 5 µm; mobile phase: [water (NH₄HCO₃) - ACN]; B%: 48% to 78%, 2 minutes), so as to obtain **Compound AB29572** (6.39 mg, 12.7 µmol, yield: 6.61%, purity: 91.3%) as a yellow solid. ¹H NMR (400 MHz, DMSO-d₆) δ ppm 12.45 (br s, 1 H), 11.44 (br s, 1 H), 7.38 - 7.59 (m, 2 H), 7.35 (s, 1 H), 7.08 (br d, *J* = 8.50 Hz, 1 H), 6.96 (s, 1 H), 2.55 (s, 3 H), 2.42 (s, 3 H), 2.07 (s, 3 H). LCMS: Ms: M +H⁺ = 460.

### Example 19 Synthesis of Compound AB29540

### (1) Synthesis of Compound 2

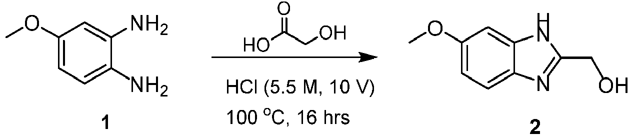

Hydrochloric acid (15 mL, 5.5 M) was added to **Compound 1** (0.7 g, 5.07 mmol, 1 eq), and glycolic acid (424 mg, 5.57 mmol, 339 µL, 1.10 eq) was added to the reaction solution, which was stirred at 100°C for 16 hours. LCMS showed that the raw materials were consumed, and the peak of the target product was detected. Saturated aqueous sodium bicarbonate solution was slowly added to the reaction solution to adjust the pH to 7 to 8, followed by extraction twice with ethyl acetate (50 mL × 2). The organic phases were combined, dried and concentrated. The crude product was purified by column chromatography (ethyl acetate: methanol = 1: 1), so as to obtain **Compound 2** (160 mg, 808 µmol, yield: 15.9%, purity: 90%) as a brown solid (LCMS: Ms: M +H⁺ = 179.4).

### (2) Synthesis of Compound 3

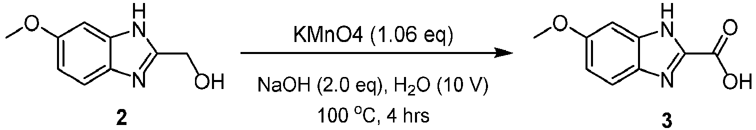

**Compound 2** (160 mg, 898 µmol, 1.00 eq) was added with a mixture of sodium hydroxide (71.8 mg, 1.80 mmol, 2.00 eq) and water (2 mL), and the reaction solution was stirred at 100°C for 2 hours. After the reaction solution was cooled to room temperature, potassium permanganate (150 mg, 949 µmol, 1.06 eq) was added thereto, and the reaction solution was heated to 100°C and then stirred for 2 hours. LCMS showed that the raw materials were consumed, and the main peak of the target product was detected. After the completion of the reaction, the reaction solution was cooled to room temperature and filtered, and the pH of the filtrate was adjusted to 4 to 5 with hydrochloric acid (2N). The resulting solution was filtered, the filter cake was washed once with water, and the filter cake was collected and dried under vacuum so as to obtain **Compound 3** (0.1 g, crude) as a red solid (LCMS: Ms: M +H⁺ = 193.3).

### (3) Synthesis of Compound 6

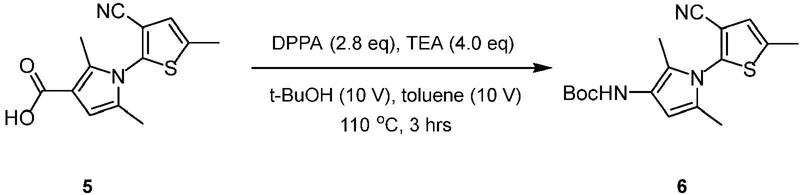

Toluene (5 mL) was added to **Compound 5** (500 mg, 1.92 mmol, 1.00 eq), and DPPA (1.48 g, 5.38 mmol, 1.17 ml, 2.80 eq) and triethylamine (777 mg, 7.68 mmol, 1.07 mL, 4.00 eq) were added to the reaction solution, and the resulting mixture was stirred at 110°C for 1 hour. Tert-butanol (5 mL) was added to the reaction solution, and the resulting mixture was stirred at 110°C for 2 hours. LCMS showed that the target product was generated. Water (10 mL) was added to the reaction solution, which was extracted twice with ethyl acetate (15 mL × 2), and the organic phases were combined and concentrated. The crude product was separated and purified by thin layer chromatography (petroleum ether: ethyl acetate = 5: 1) so as to obtain **Compound 6** (50.0 mg, 105 µmol, yield: 5.50%, purity: 70%) as a yellow oil.

### (4) Synthesis of Compound 7

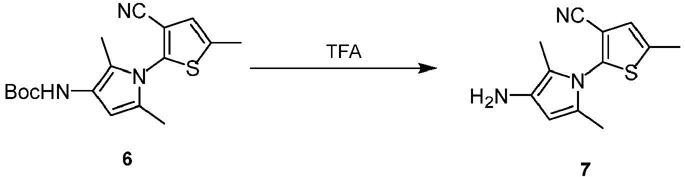

Compound **6** (30.0 mg, 90.5 µmol, 1.00 eq) was added with a mixed solution of trifluoroacetic acid (0.1 mL) and dichloromethane (2 mL), and the reaction solution was stirred at 20°C for 16 hours. LCMS showed that the raw materials were consumed, and the main peak of the target product was detected. The reaction solution was directly concentrated, so as to obtain **Compound 7** (0.04 g, crude, TFA) as a light yellow oil, which was directly used in the next reaction.

### (5) Synthesis of Compound AB29540

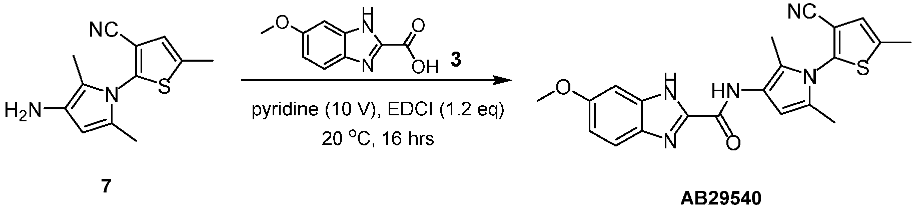

Pyridine (1 mL) was added to **Compound 3** (20.0 mg, 104 µmol, 1.00 eq), and Compound **7** (35.9 mg, 104 µmol, 1.00 eq, TFA) and EDCI (21.9 mg, 114 µmol, 1.10 eq) were added to the reaction solution, which was stirred at 20°C for 16 hours. LCMS showed that the raw materials were consumed, and the peak of the target product was detected. The reaction solution was directly concentrated. The crude product was separated and purified by high performance liquid chromatography (column: Waters xbridge 150 × 25mm 10 µm; mobile phase: [water (NH₄HCO₃) - ACN]; B%: 38% to 68%, 9 minutes), so as to obtain **Compound AB29540** (3.08 mg, 7.33 µmol, yield: 7.05% , purity: 96.53%). ¹H NMR (400 MHz, DMSO-*d*₆) *δ* ppm 13.17 (dt, *J* = 4.41,2.11 Hz, 1 H), 10.06 (s, 1 H), 7.45 - 7.73 (m, 1 H), 7.29 (s, 1 H), 6.87 - 7.13 (m, 2 H), 6.26 (s, 1 H), 3.82 (s, 3 H), 2.53 (br s, 3 H), 2.08 (s, 3 H), 2.04 (s, 3 H). LCMS: Ms: M +H⁺ = 406.

### Example 20 Synthesis of Compound AB29554

### (1) Synthesis of Compound 2

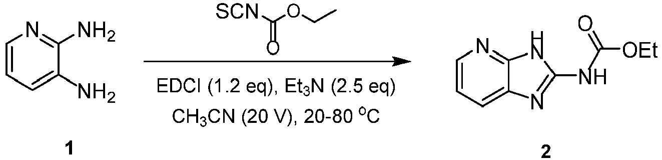

Acetonitrile (20.0 mL) was added to **Compound 1** (500 mg, 4.58 mmol, 1.00 eq), to which triethylamine (463 mg, 4.58 mmol, 638 µL, 1.00 eq) was added, and then ethoxycarbonyl isothiocyanate (661 mg, 5.04 mmol, 1.10 eq) was added dropwise to the reaction solution, which was stirred at25°C for 15 minutes. 1-(3-dimethylaminopropyl)-3-ethylcarbodiimide (1.05 g, 5.50 mmol, 1.20 eq) was added to the reaction solution, and then triethylamine (695 mg, 6.87 mmol, 956 µL, 1.50 eq) was added to the reaction solution, and the reaction mixture was stirred at 80°C for 3 hours. The reaction solution was filtered, the filter cake was washed once with a small amount of acetonitrile (10.0 mL), and the filter cake was collected so as to obtain **Compound 2** (900 mg, crude) as a gray solid.

### (2) Synthesis of Compound 3

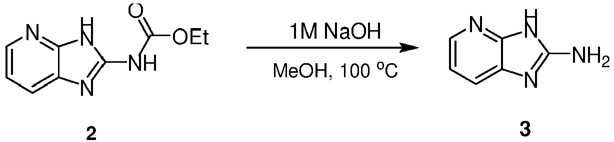

Methanol (4.00 mL) was added to **Compound 2** (900 mg, 4.36 mmol, 1.00 eq), and sodium hydroxide solution (1 M, 8.73 mL, 2.00 eq) was added to the reaction solution, which was stirred at 100°C for 16 hours. LCMS showed that the raw materials were consumed, and the peak of the target product was detected. After the reaction solution was cooled, methanol was removed, and 2M HCl solution was slowly added until the pH of the resulting solution was 7 to 8. A solid was precipitated and was directly filtered, and the filter cake was collected, so as to obtain **Compound 3** (500 mg, 3.73 mmol, yield: 85.4% ) as a gray solid.

### (3) Synthesis of Compound 6

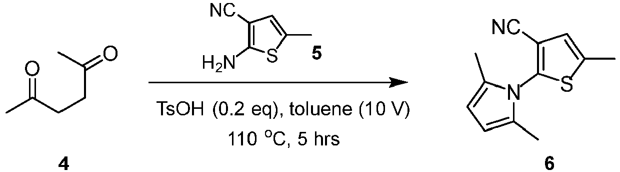

Toluene (2 mL) was added to Compound **4** (200 mg, 1.75 mmol, 205 µL, 1.00 eq), and Compound **5** (242 mg, 1.75 mmol, 1.00 eq) and anhydrous p-toluenesulfonic acid (60.3 mg, 350 µmol, 0.200 eq) were added to the reaction solution, which was stirred at 110°C for 4 hours. TLC (petroleum ether: ethyl acetate = 5: 1, Rf = 0.43) showed that a small amount of Compound 5 remained, and a main spot with lower polarity was generated. Water (10 mL) was added to the reaction solution, which was extracted twice with ethyl acetate (10 mL × 2), and the organic phases were combined and concentrated. The crude product was separated and purified by thin layer chromatography (petroleum ether: ethyl acetate = 5: 1), so as to obtain Compound **6** (215 mg, 994 µmol, yield: 56.7%) as a brown solid. ¹H NMR (400 MHz, CDCl₃) δ ppm 6.88 (d, *J* = 1.00 Hz, 1 H), 5.93 (s, 2 H), 2.52 (d, *J* = 1.00 Hz, 3 H), 2.12 (s, 6 H). LCMS: Ms: M +H⁺ = 217.

### (4) Synthesis of Compound 7

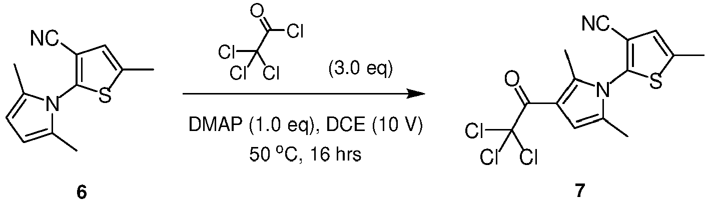

1,2-Dichloroethane (2 mL) was added to Compound **6** (100 mg, 462 µmol, 1.00 eq), to which 4-dimethylaminopyridine (56.5 mg, 462 µmol, 1.00 eq) was added, and trichloroacetyl chloride (252 mg, 1.39 mmol, 154 µL, 3.00 eq) was added dropwise to the reaction solution, which was stirred at 50°C for 16 hours. TLC (petroleum ether: ethyl acetate = 5: 1, Rf = 0.24) showed that about 20% of the raw materials were not completely reacted, and a new spot with higher polarity was detected. Water (10 mL) was added to the reaction solution, which was extracted twice with ethyl acetate (10 mL × 2), and the organic phases were combined, dried and concentrated. The crude product was separated and purified by thin layer chromatography (petroleum ether: ethyl acetate = 5: 1), so as to obtain **Compound 7** (80.0 mg, 221 µmol, yield: 47.8%) as a light yellow oil. ¹H NMR (400 MHz, CDCl₃) δ ppm 6.97 (d, *J* = 0.75 Hz, 1 H), 6.73 (s, 1 H), 2.57 (d, *J* = 0.75 Hz, 3 H), 2.47 (s, 3 H), 2.12 (s, 3 H).

### (5) Synthesis of Compound AB29554

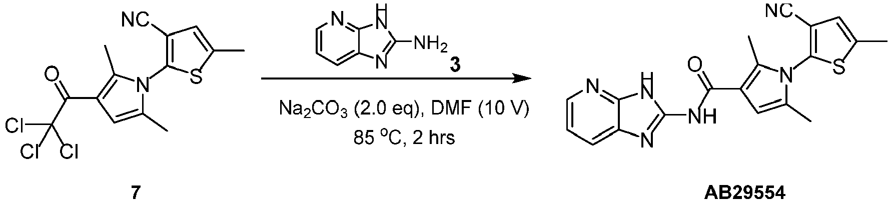

N,N-dimethylformamide (2 mL) was added to Compound 7 (60.0 mg, 166 µmol, 1.00 eq), and Compound 3 (26.7 mg, 199 µmol, 1.20 eq) and anhydrous sodium carbonate (35.2 mg, 332 µmol, 2.00 eq) were added to the reaction solution, which was stirred at 85°C for 2 hours. LCMS showed that the raw materials were consumed, and the main peak of the target product was detected. The reaction solution was directly concentrated and dried. The crude product was separated and purified by high performance liquid chromatography (column: Waters xbridge 150 × 25mm 10 µm; mobile phase: [water (NH₄HCO₃) - ACN]; B%: 27% to 57%, 9 minutes), so as to obtain **Compound AB29554** (20.0 mg, 52.4 µmol, yield: 31.6%, purity: 98.66%) as a white solid. ¹H NMR (400 MHz, DMSO-d₆) *δ* ppm 12.27 - 12.45 (m, 1 H), 11.41 - 11.63 (m, 1 H), 8.16 - 8.28 (m, 1 H), 7.78 (br d, *J* = 8.00 Hz, 1 H), 7.35 (d, *J* = 1.13 Hz, 1 H), 7.09 (br d, *J* = 4.38 Hz, 1 H), 6.91 - 7.01 (m, 1 H), 2.56 (s, 3 H), 2.42 (s, 3 H), 2.08 (s, 3 H). LCMS: Ms: M +H⁺ = 377.

### Example 21 Synthesis of Compound AB29566

### (1) Synthesis of Compound 2

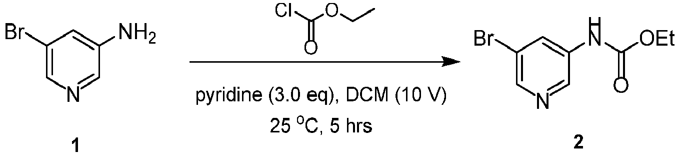

**Compound 1** (5.00 g, 28.9 mmol, 1.00 eq) was dissolved in dichloromethane (50.0 mL), and ethyl chloroformate (3.36 g, 30.9 mmol, 2.95 mL, 1.07 eq) was added thereto, then pyridine (6.86 g, 86.7 mmol, 7.00 mL, 3.00 eq) was slowly added dropwise into the above reaction solution. The temperature was controlled at 25°C to 30°C, and the system was stirred at 25°C for 1.5 hours. LCMS showed that **Compound 1** was consumed, and the molecular weight of the desired compound was detected. After the completion of the reaction, the reaction solution was slowly poured into ice water (100 mL) and stirred for quenching. After quenching, dichloromethane (100 mL × 3) was added for extraction, and the organic phase was washed with saturated saline solution (50.0 mL), dried over anhydrous sodium sulfate and then concentrated under reduced pressure so as to obtain **Compound 2** (5.00 g, crude product) as a yellow solid (LCMS: Ms: M +H⁺ = 245.0).

### (2) Synthesis of Compound 3

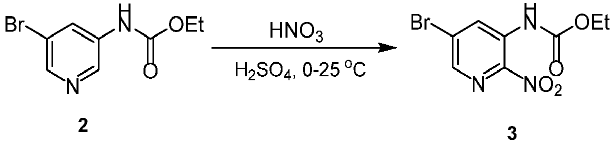

Fuming nitric acid (1.08 g, 17.1 mmol, 771 µL, 1.00 eq) was dissolved in sulfuric acid (12.0 mL), and **Compound 2** (4.20 g, 17.1 mmol, 1.00 eq) was slowly added to the above mixed solution at 0°C. After being stirred at 0°C for 0.1 hour, the reaction solution was slowly heated to 25°C and stirred for 12 hours. LCMS showed that **Compound 2** was consumed, and the molecular weight of the desired compound was detected. The reaction solution was poured into ice water, a solid was precipitated and filtered under vacuum, and the filter cake was collect and concentrated under reduced pressure so as to obtain **Compound 3** (4.30 g, crude product) as a white solid (LCMS: Ms: M +H⁺ = 291.9).

### (3) Synthesis of Compound 4

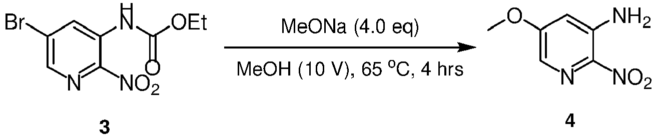

**Compound 3** (2.50 g, 8.62 mmol, 1.00 eq) was dissolved in methanol (10.0 mL), and then sodium methoxide (6.21 g, 34.4 mmol, purity: 30%, 4.00 eq) was added. The resulting mixture was stirred at 65°C for 12 hours. LCMS showed that **Compound 3** was consumed, and the molecular weight of the desired compound was detected. The reaction solution was concentrated under vacuum, to which water (20.0 mL) was added, and the resulting solution was filtered under vacuum. The filter cake was collected and concentrated under reduced pressure so as to obtain **Compound 4** (900 mg, crude product) as a gray solid (LCMS: Ms: M +H⁺ = 170.1).

### (4) Synthesis of Compound 5

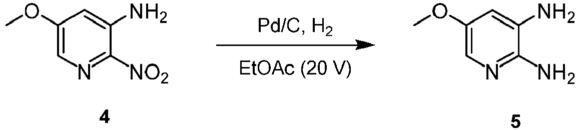

**Compound 4** (1.19 g, 7.04 mmol, 1.00 eq) was dissolved in ethyl acetate (100 mL), and wet palladium/carbon (0.50 g, purity: 10%) was added thereto. The resulting mixture was purged with hydrogen for three times. The reaction solution was stirred at 25°C and 15 Psi for 12 hours. LCMS showed that **Compound 4** was consumed, and the molecular weight of the desired compound was detected. The reaction solution was filtered under vacuum, and the filtrate was collected and concentrated under reduced pressure so as to obtain **Compound 5** (800 mg, crude product) as a brown oil (LCMS: Ms: M +H⁺ = 140.2).

### (5) Synthesis of Compound 6

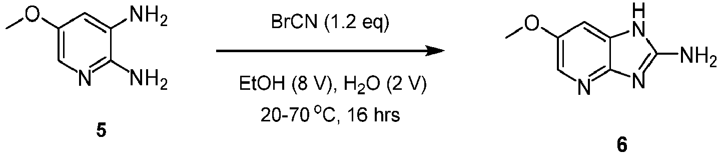

**Compound 5** (800 mg, 5.75 mmol, 1.00 eq) was dissolved in water (2.00 ml), ethanol (8.00 mL) was added thereto. The temperature of the resulting solution was lowered to 0°C, and then hydrogen bromide (730 mg, 6.90 mmol, 507 µL, 1.20 eq) was slowly added. The resulting mixture was heated to 70°C and reacted for 12 hours. LCMS showed that 20% of Compound 5 remained, and the molecular weight of the desired compound was detected. The reaction solution was concentrated under vacuum. After purification by TLC (ethyl acetate: methanol = 10: 1), **Compound 6** (400 mg, 2.44 mmol, yield: 42.3%) was obtained as a gray solid (LCMS: Ms: M +H⁺ = 165.2).

### (6) Synthesis of Compound AB29566

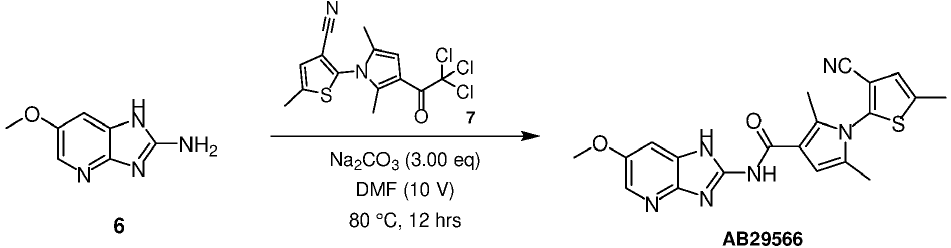

**Compound 6** (50.0 mg, 138 µmol, 1.00 eq) was dissolved in N,N-dimethylformamide (2.00 mL), and then sodium carbonate (43.96 mg, 414 µmol, 3.00 eq) and **Compound 7** (22.7 mg, 138 µmol, 1.00 eq) were added thereto. The mixture was stirred at 80°C for 2 hours. LCMS showed that the raw materials were consumed, and the molecular weight of the desired compound was detected. The reaction solution was concentrated under vacuum and purified by high performance liquid chromatography (column: Welch Xtimate C18 150 × 25 mm × 5 µm; mobile phase: [water (NH₃H₂O) - ACN]; B%: 27 % to 57%, 8 minutes), so as to obtain **Compound AB29566** (10.7 mg, 26.4 µmol, 19.1% yield) as a yellow solid. ¹H NMR (400 MHz, DMSO-d6) *δ* ppm 7.74 - 7.96 (m, 1 H) 7.32 (br d, *J* = 1.13 Hz, 2 H ) 6.72 - 6.90 (m, 1 H) 3.80 (s, 3 H) 2.53 (s, 3 H) 2.41 (s, 3 H) 2.05 (s, 3 H). LCMS: Ms: M +H⁺ = 407.

### Example 22: Synthesis of Compound AB29552

### (1) Synthesis of Compound 4

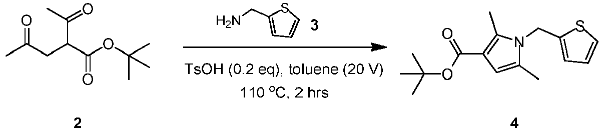

Toluene (2 mL) was added to Compound **2** (100 mg, 467 µmol, 1.00 eq), and Compound **3** (52.8 mg, 467 µmol, 48.0 µL, 1.00 eq) and anhydrous p-toluenesulfonic acid (16.0 mg, 93.3 µmol, 0.200 eq) were added to the reaction solution, which was stirred at 110°C for 2 hours. LCMS showed that the raw materials were consumed, and the main peak of the target product was detected. The reaction solution was directly concentrated. The crude product was separated and purified by thin layer chromatography (petroleum ether: ethyl acetate = 3: 1), so as to obtain Compound **4** (110 mg, 377 µmol, 80.9% yield) as a brown solid.

### (2) Synthesis of Compound 5

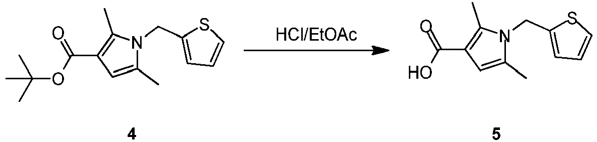

HCl/EtOAc (2 mL, 4M) was added to Compound **4** (110 mg, 377 µmol, 1.00 eq), and the reaction solution was stirred at 20°C for 3 hours. TLC (petroleum ether: ethyl acetate = 3:1, Rf = 0.24) showed that the raw materials were consumed, and that a new spot with higher polarity was generated. The reaction solution was directly concentrated, so as to obtain Compound **5** (0.07 g, crude) as a brown solid, which was directly used in the next reaction.

### (3) Synthesis of Compound AB29552

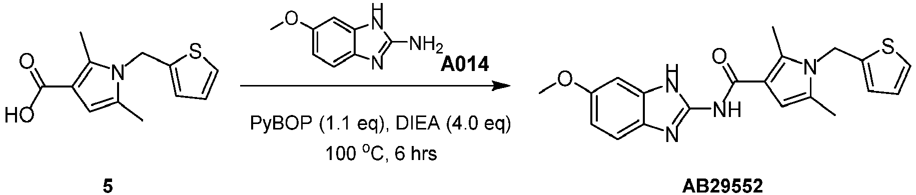

N,N-dimethylformamide (1 mL) was added to Compound 5 (60.0 mg, 255 µmol, 1.00 eq), and Compound **A014** (45.8 mg, 280 µmol, 1.10 eq), benzotriazol-1-yl-oxytripyrrolidinophosphonium hexafluorophosphate (146 mg, 280 µmol, 1.10 eq) and N,N-diisopropylethylamine (132mg, 1.02 mmol, 178 µL, 4.00 eq) were added to the reaction solution, which was stirred at 100°C for 6 hours. LCMS showed that the raw materials were completely reacted and the target product was detected. The reaction solution was directly concentrated. The crude product was separated and purified by high performance liquid chromatography (column: Welch Xtimate C18 150 × 25 mm × 5 µm; mobile phase: [water (HCl) - ACN]; B%: 22% to 52%, 10 minutes), followed by secondary purification (column: Waters xbridge 150 × 25 mm 10µm; mobile phase: [water (NH₄HCO₃) - ACN]; B%: 40% to 70%, 9 minutes), so as to obtain **Compound AB29552** (2.34 mg, 6.15 µmol, yield: 2.41%) as a white solid. ¹H NMR (400 MHz, DMSO-*d*₆) δ ppm 12.00 (br d, *J* = 0.75 Hz, 1 H), 10.84 - 10.96 (m, 1 H), 7.46 (dd, *J* = 5.07, 1.19 Hz, 1 H), 7.24 - 7.37 (m, 1 H), 6.89 - 7.09 (m, 3 H), 6.62 - 6.80 (m, 2 H), 5.32 (s, 2 H), 3.75 (s, 3 H), 2.60 (s, 3 H), 2.22 (s, 3 H). LCMS: Ms: M +H⁺ = 381.

### Example 23 Synthesis of Compound AB29555

### (1) Synthesis of Compound 2

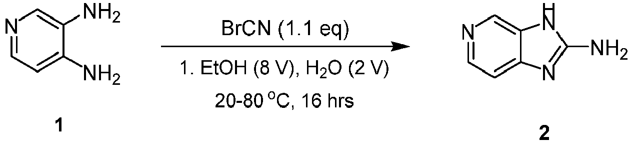

Compound **1** (1.00 g, 9.16 mmol, 1.00 eq) was dissolved in ethanol (2.00 mL) and water (10.0 mL), and hydrogen bromide (1.16 g, 11.0 mmol, 808 µL, 1.20 eq) was slowly added thereto. The reaction mixture was stirred at 70°C for 12 hours. LCMS showed that 30% of Compound **1** remained, and the molecular weight of the desired compound was detected. The reaction mixture was concentrated under vacuum so as to obtain Compound **2** (2.00 g, crude product) as a yellow solid (LCMS: Ms: M +H⁺ = 130.4).

### (2) Synthesis of Compound AB29555

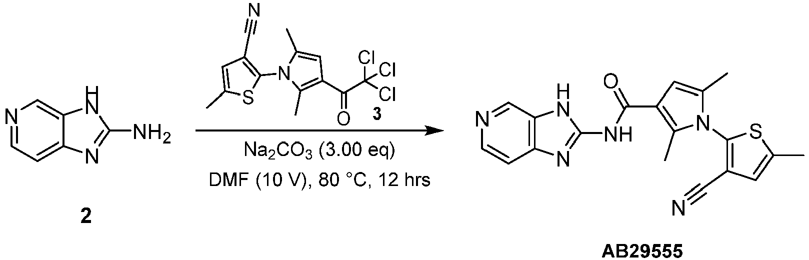

**Compound 2** (29.6 mg, 220 µmol, 2.00 eq) was dissolved in N,N-dimethylformamide (2.00 mL), and sodium carbonate (35.1 mg, 331 µmol, 3.00 eq) and **Compound 3** (40.0 mg, 110 µmol, 1.00 eq) were added thereto. The resulting mixture was stirred at 80°C for 12 hours. LCMS showed that the raw materials were consumed, and the molecular weight of the desired compound was detected. The reaction solution was concentrated under vacuum. The crude product was purified by high performance liquid chromatography (column: Welch Xtimate C18 150 × 25mm × 5 µm; mobile phase: [water (HCl) - ACN]; B%: 14% to 44%, 8 minutes), so as to obtain **Compound AB29555** (10.0 mg, 24.2 µmol, yield: 21.9%, hydrochloric acid) as a yellow solid. ¹H NMR (400 MHz, MeOD) δ ppm 8.88 (s, 1 H) 8.32 - 8.49 (m, 1 H) 7.80 - 8.02 (m, 1 H) 7.16 (d, *J* = 1.00 Hz, 1 H) 6.71 (s, 1 H) 2.59 (s, 3 H) 2.48 (s, 3 H) 2.15 (s, 3 H)_{∘} LCMS: Ms: M+H⁺=377.

### Example 24 Synthesis of Compound AB29573

### (1) Synthesis of Compound 2

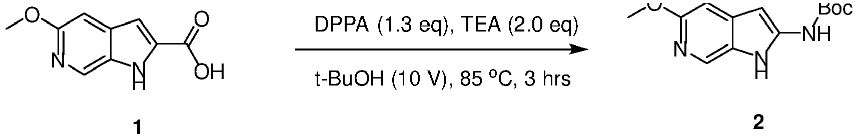

Tert-butanol (5 mL) was added to **Compound 1** (500 mg, 2.54 mmol, 1.00 eq), and triethylamine (643 mg, 6.36 mmol, 885 µL, 2.50 eq) and DPPA (910 mg, 3.31 mmol, 716 µl, 1.30 eq) were added to the reaction solution, which was stirred at 85°C for 2 hours. LCMS showed that the raw materials were consumed, and the main peak of the target product was detected. The reaction solution was directly concentrated. The crude product was separated and purified by silica gel column (petroleum ether: ethyl acetate = 10: 1 to 1: 1), so as to obtain **Compound 2** (100 mg, 374 µmol, yield: 14.7%) as a white solid.

### (2) Synthesis of Compound 3

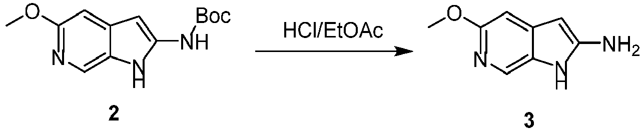

HCl/EtOAc (7 mL) was added to **Compound 2** (75.0 mg, 285 µmol, 1.00 eq), and the reaction solution was stirred at 20°C for 2 hours. LCMS showed that the raw materials were consumed, and the peak of the target product was detected. The reaction solution was directly concentrated so as to obtain **Compound 3** (0.06 g, crude, HCl) as a yellow solid, which was directly used in the next reaction.

### (3) Synthesis of Compound AB29573

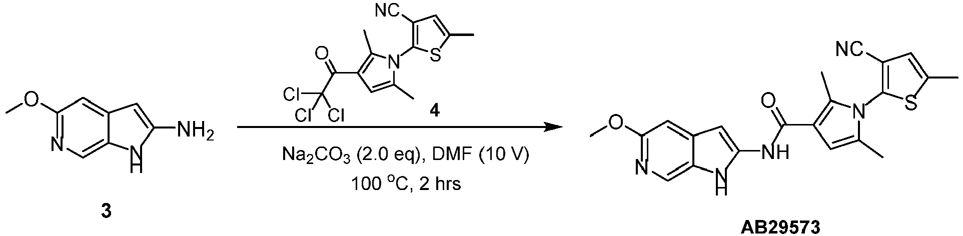

N,N-dimethylformamide (1 mL) was added to **Compound 4** (50.0 mg, 138 µmol, 1.00 eq), and **Compound 3** (55.2 mg, 276 µmol, 2.00 eq, HCl) and anhydrous sodium carbonate (58.6 mg, 553 µmol, 4.00 eq) were added to the reaction solution, which was stirred at 100°C for 2 hours. LCMS showed that the raw materials were completely reacted, and a small amount of the target product was detected. After the reaction solution was concentrated, methanol was added thereto, followed by filtration, and the filtrate was collected and concentrated. The crude product was separated and purified by thin layer chromatography (petroleum ether: ethyl acetate = 1: 1) and then by high performance liquid chromatography (column: Welch Xtimate C18 150 × 25 mm × 5 µm; mobile phase: [water (NH₃H₂O) - ACN]; B%: 30% to 60%, 8 minutes), so as to obtain **Compound AB29573** (1 mg, 2.30 µmol, yield: 1.66%, purity: 93.06% ) as a yellow solid. ¹H NMR (400 MHz, DMSO-d₆) δ ppm 11.17 - 11.40 (m, 1 H), 10.58 - 10.74 (m, 1 H), 8.25 (s, 1 H), 7.34 (d, *J* = 1.13 Hz, 1 H), 6.73 (s, 1 H), 6.65 (s, 1 H), 6.06 (s, 1 H), 3.79 (s, 3 H), 2.54 (d, *J* = 0.63 Hz, 3 H), 2.38 (s, 3 H), 2.09 (s, 3 H). LCMS: Ms: M +H⁺ = 406.

### Pharmacological activity test and analysis of results

### Preparation of recombinant DHX33

Regarding the isolation and purification of proteins, please refer to Wang X, Ge W, and Zhang Y. Recombinant DHX33 Protein Possesses Dual DNA/RNA Helicase Activity. Biochemistry. 2019;58 (4):250-8. RNA helicase gene (mouse DHX33 gene) was cloned into a pET32M-3C vector (between BamH I/Not I restriction sites). Afterward, the plasmid was transformed into BL-21pLysS (DE3) E. coli. 0.5 mM isopropyl 1-thio-β-D-galactopyranoside (IPTG) was added, and the expression of the recombinant protein was induced at 16°C for 16 hours. Cells were allowed to precipitate and were resuspended in a cell lysis buffer [50 mM Tris-HCl (pH7.2), 150 mM NaCl, 1%Triton X-100, and 50 mM imidazole supplemented with a protease inhibitor]. Thereafter, cells were subjected to ultrasonic treatment and centrifuged at a rotation speed of 13000 rpm for 25 minutes. The supernatant was incubated with the nickel-nitrilotriacetic acid beads equilibrated in Tris buffer, followed by sufficient washing. Thereafter, the purified protein was eluted with a solution of 300 mM imidazole in Tris buffer, and was then subjected to dialysis against an imidazole-free Tris buffer at 4°C overnight.

Fig. 1 showed the analysis of the results obtained after the recombinant DHX33 protein prepared by the above-mentioned method was separated by SDS-PAGE and then stained with Coomassie brilliant blue. The arrow in the figure indicated the target recombinant DHX33 protein (containing a thioredoxin tag), and the molecular weight of the target band was 90 kDa.

### Analysis of the helicase activity of DHX33

The components involved in the reaction for testing the helicase activity were added to a 96-well opaque white plate. The method was outlined as follows. The 96-well plate was coated with neutravidin at a final concentration of 10 µg/mL (100 µL/well) overnight at 4°C. Afterwards, the neutravidin-coated plate was blocked with 100 µL of 0.1% (w/v) BSA (dissolved in normal PBS) at 22°C for 2 hours. After washing, a DNA duplex (2.5 ng) that was resulted from the annealing of two single-stranded DNA oligonucleotides (the sequence of one single strand was biotin-labeled 5'-GCTGACCCTGCTCCCAATCGTAATCTATAG-3'; and the sequence of the other single strand was DIG-labeled 5'-CGATTGGGAGCAGGGTCAGC-3') [the annealing reaction was carried out in PBS (pH 7.0) containing 1M NaCl] was added, and the resulting mixture was incubated at 22°C for 4 hours. After the addition of 90 µL of the reaction mixture, the helicase reaction was initiated [0.25 µg of purified full-length DHX33 protein, dissolved in 25 mM 4-MOPS (pH 7.0), 5 mM ATP, 2 mM DTT, 3 mM MnCl₂ and 100 µg/mL BSA]. The reaction was conducted at 37°C for 60 minutes. After being washed, each well was incubated with a blocking solution [10% (w/v) BSA in 0.1 M maleic acid and 0.15 M NaCl (pH 7.5)] for 30 minutes, and was then incubated with 20 µL of antibody solution (anti-DIG-AP, Roche, in blocking buffer) for 30 minutes. After being washed with 100 µL of detection buffer [0.1 M Tris-HCl and 0.1 M NaCl (pH 9.5)], each well was added with 1 µL of chemiluminescent substrate (CSPD-0.25 mM), and the plate was incubated at 17°C for 5 minutes. Afterwards, the plate was patted dry and incubated at 37°C for 30 minutes. The remaining DIG-AP marker control in each well was counted for 10 minutes by a luminescence multi-well plate reader (Enspire, PerkinElmer). Higher reading indicated weaker helicase activity. Positive control and negative controls were set for this acticity assay. Among them, one negative control group, which differed from the experimental group only in that the former was not added with ATP (other conditions were the same as the experimental group), was used for the comparison with the experimental group; another negative control group, which differed from the experimental group only in that the former was not added with DHX33 protein (other conditions were the same as the experimental group), was used for the comparison with the experimental group. The positive control group was set for the comparison with the wild-type DHX33 protein (standard substance). The results obtained from the comparison with DHX33 protein (standard substance) indicated that DHX33 protein prepared by the above-mentioned method had helicase activity.

Regarding the specific analytical method of helicase activity, please refer to Wang X, Ge W, and Zhang Y. Recombinant DHX33 Protein Possesses Dual DNA/RNA Helicase Activity. Biochemistry. 2019;58 (4):250-8.

### Inhibition of the helicase activity of DHX33 by compounds

Compounds having a series of concentration (the concentrations were set at 1 nM, 5 nM, 10 nM, 20 nM, 50 nM, 100 nM, 250 nM, 500 nM, and 1000 nM) were used to conduct the helicase activity assay of DHX33 protein *in vitro.* The half-maximal inhibitory concentrations of the compounds of the present disclosure on the helicase activity of DHX33 protein were as shown in Table 1. As could be seen from Table 1, the compounds of the present disclosure had significant inhibitory effects on the helicase activity of DHX33 protein.

**Table 1: Assay of the inhibitory effects of the compounds on the helicase activity of DHX33 protein**

| Compound | Structure | IC₅₀ |
|---|---|---|
| Compound as control | 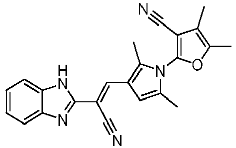 | * |
| AB24386 | 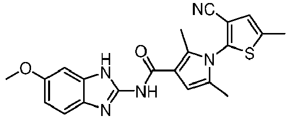 | **** |
| AB24387 | 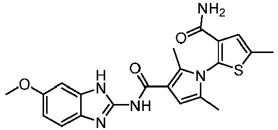 | *** |
| AB29501 | 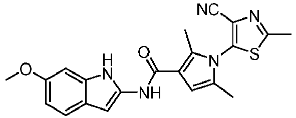 | **** |
| AB29556 | 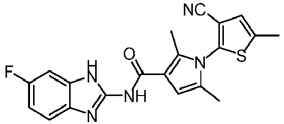 | *** |
| AB29558 | 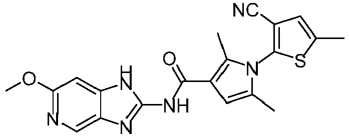 | **** |
| AB29559 | 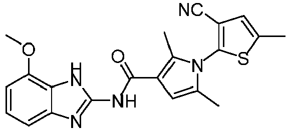 | * |

| | | |
|---|---|---|
| * denoted that the half-maximal inhibitory concentration was 400 nM or more; ** denoted that the half-maximal inhibitory concentration was 100 nM or more and less than 400 nM; *** denoted that the half-maximal inhibitory concentration was 20 nM or more and less than 100 nM; **** denoted that the half-maximal inhibitory concentration was less than 20 nM. | | |

### Cell culture and sources

U251-MG cells were purchased from the cell bank of the Chinese Academy of Sciences. Cells were cultured in MEM medium containing 10% fetal bovine serum (FBS), 2 mM L-glutamine, streptomycin and penicillin. The growth conditions were set as follows: a humidified cell incubator (37°C, 5% CO₂). Cells were passaged every 3 days and were discarded after 10 passages.

### Determination of half-maximal cell-inhibitory concentrations (IC₅₀)

U251-MG cells (a DHX33-overexpressing cancer cell strain) were seeded on a 96-well plate at 1 × 10⁴ cells/100µl/well. After the cells were completely attached, the compounds were added to the cell culture medium at a concentration of 5 nM, 10 nM, 25 nM, 50 nM, 100 nM, 250 nM, 500 nM, 1000 nM, 2000 nM, 5000 nM, 10 µM or 20 µM, and the resulting mixture was uniformly mixed with a multichannel pipette. After the cells were incubated with the compound for 48 hours, CCK-8 reagent (Yeasen Biotechnology (Shanghai) Co., Ltd.) was added to the medium in the 96-well plate in accordance with the standard procedure. After being incubated for 2 hours, the plate was read with a microplate reader (OD = 450nm). The experiment was repeated three times, the inhibiton curve of the compound at different concentrations was plotted, and the half-maximal inhibitory concentration (IC₅₀) of the compound was calculated.

**Table 2: Determination of the half-maximal inhibitory concentrations of compounds on U251-MG cell**

| Compound | Structure | IC₅₀ |
|---|---|---|
| Compound as control | 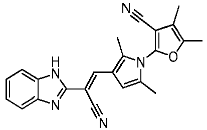 | * |
| AB24386 | 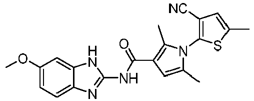 | **** |
| AB24387 | 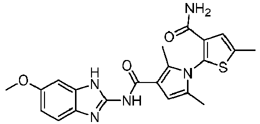 | *** |
| AB29501 | 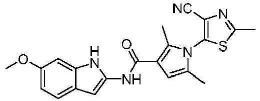 | **** |
| AB29502 | 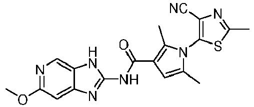 | *** |
| AB29505 | 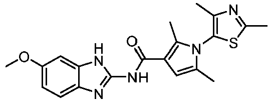 | *** |
| AB29509 | 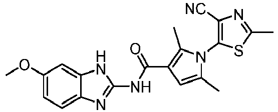 | *** |
| AB29510 | 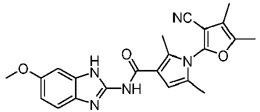 | *** |
| AB29513 | 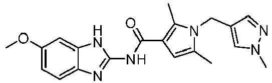 | * |
| AB29522 | 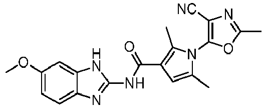 | *** |
| AB29553 | 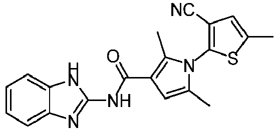 | *** |
| AB29556 | 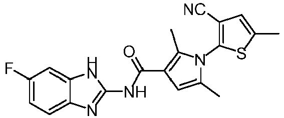 | *** |
| AB29557 | 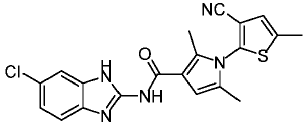 | *** |
| AB29558 | 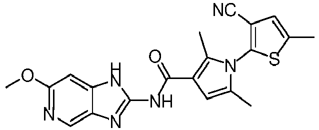 | **** |
| AB29559 | 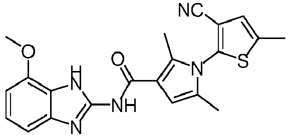 | ** |
| AB29560 | 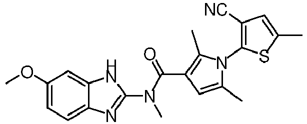 | *** |
| AB29561 | 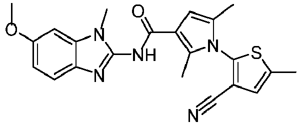 | *** |
| AB29562 | 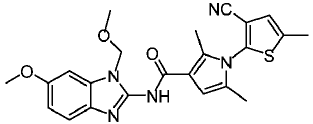 | *** |
| AB29563 | 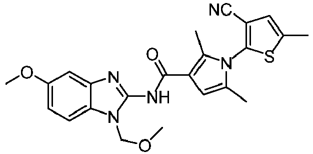 | *** |
| AB29564 | 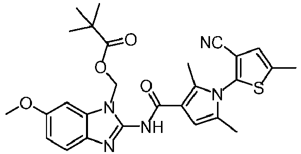 | ** |
| AB29565 | 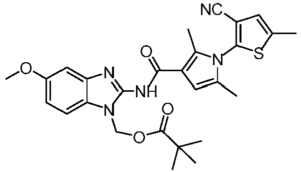 | ** |
| AB29572 | 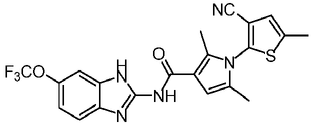 | ** |
| AB29552 | 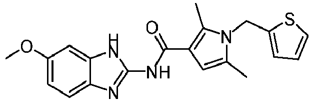 | ** |
| AB29566 | 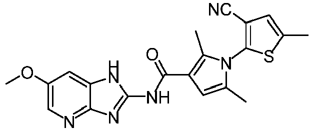 | **** |
| AB29554 | 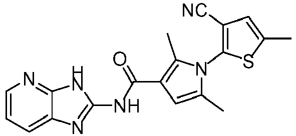 | ** |
| AB29540 | 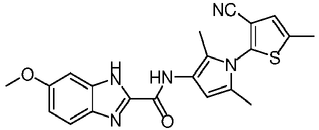 | *** |
| AB29573 | 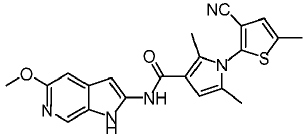 | *** |
| AB29555 | 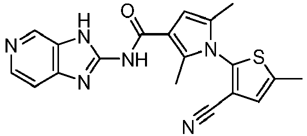 | ** |

| | | |
|---|---|---|
| * denoted that the half-maximal inhibitory concentration was 5 µM or more and less than 20 µM; ** denoted that the half-maximal inhibitory concentration was 1 µM or more and less than 5 µM; *** denoted that the half-maximal inhibitory concentration was 100 nM or more and less than 1000 nM; **** denoted that the half-maximal inhibitory concentration was less than 100 nM. | | |

As could be seen from Table 2, the compounds of the present disclosure had significant inhibitory effects on U251-MG cells (a DHX33-overexpressing cancer cell strain).

## Claims

1. A compound having the structure of formula I or a pharmaceutically acceptable form thereof: 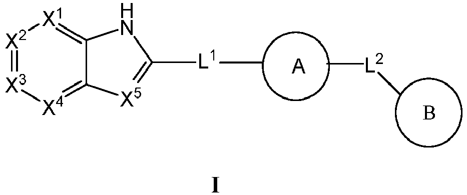 wherein
X¹ is N or CR¹, X² is N or CR², X³ is N or CR³, X⁴ is N or CR⁴;
R¹, R², R³ and R⁴ are each independently hydrogen, halogen, amino, nitro, hydroxyl, C₁₋₆ alkyl, C₁₋₆ haloalkyl, C₁₋₆ alkoxy, C₁₋₆ haloalkoxy, -NH(C₁₋₆ alkyl), -N(C₁₋₆ alkyl)₂, C₁₋₆ hydroxyalkyl, -O-(C₁₋₆ alkylene)-O-(C₁₋₆ alkyl), -C(=O)-NH-(C₁₋₆ alkyl), -C(=O)-NH-(C₁₋₆ alkylene)-N(C₁₋₆ alkyl)₂, or -C(=O)-O-(C₁₋₆ alkyl);
X⁵ is N or CR⁵, R⁵ is hydrogen, halogen, or C₁₋₆ alkyl, and the C₁₋₆ alkyl is optionally substituted with one or more substituent selected from halogen, C₁₋₆ alkyl, -(C₁₋₆ alkylene)-O-(C₁₋₆ alkyl) or -(C₁₋₆ alkylene)-O-C(=O)-(C₁₋₆ alkyl);
L¹ is -NR⁶C(=O)-, -NR⁶S(=O)₂-, -NR⁶S(=O)-, -C(=O)NR⁶-, -S(=O)₂NR⁶- or -S(=O)NR⁶-;
each R⁶ is independently hydrogen, C₁₋₆ alkyl, C₃₋₈ cycloalkyl, or C₆₋₁₀ aryl;
ring A is a 5-10 membered heteroaryl or a 3-8 membered heterocyclyl, ring A is optionally substituted with one or more R⁷;
each R⁷ is independently halogen, C₁₋₆ alkyl, or C₁₋₆ haloalkyl;
L² is a single bond, O, S, or CR⁸R⁹;
R⁸ and R⁹ are each independently hydrogen, halogen, or C₁₋₆ alkyl;
ring B is C₆₋₁₀ aryl, a 5-12 membered heteroaryl, or a 3-8 membered heterocyclyl, ring B is optionally substituted with one or more R¹⁰;
each R¹⁰ is independently halogen, cyano, amino, nitro, hydroxyl, C₁₋₆ alkyl, C₁₋₆ haloalkyl, C₁₋₆ alkoxy, C₁₋₆ haloalkoxy, C₃₋₆ cycloalkyl, -C(=O)-O-(C₁₋₆ alkyl), phenyl, benzyl, pyridyl, - C(=O)-NH₂, or -NH-C(=O)-(C₁₋₆ alkyl), and the phenyl, benzyl, or pyridyl is optionally substituted with one or more substituents selected from hydrogen, halogen, cyano, amino, hydroxyl, C₁₋₆ alkyl or C₁₋₆ alkoxy;
said pharmaceutically acceptable form is selected from a pharmaceutically acceptable salt, an ester, a stereoisomer, a tautomer, a solvate, a N-oxide, an isotopically labeled form, a metabolite and a prodrug.

2. The compound or the pharmaceutically acceptable form thereof according to claim 1, wherein
R¹, R², R³ and R⁴ are each independently hydrogen, halogen, amino, nitro, hydroxyl, C₁₋₆ alkyl, C₁₋₆ haloalkyl, C₁₋₆ alkoxy, C₁₋₆ haloalkoxy, -NH(C₁₋₆ alkyl), -N(C₁₋₆ alkyl)₂, or C₁₋₆ hydroxyalkyl;
preferably, R¹, R², R³ and R⁴ are each independently hydrogen, halogen, amino, nitro, hydroxyl, C₁₋₄ alkyl, C₁₋₄ haloalkyl, C₁₋₄ alkoxy, or C₁₋₄ haloalkoxy;
more preferably, R¹, R², R³ and R⁴ are each independently hydrogen, halogen, amino, nitro, hydroxyl, methyl, methoxy, or trifluoromethoxy.

3. The compound or the pharmaceutically acceptable form thereof according to claim 1 or 2, wherein
R⁵ is hydrogen, halogen, or C₁₋₄ alkyl, and the C₁₋₄ alkyl is optionally substituted with one or more substituents selected from halogen, C₁₋₄ alkyl, -(C₁₋₄ alkylene)-O-(C₁₋₄ alkyl), or -(C₁₋₄ alkylene)-O-C(=O)-(C₁₋₄ alkyl);
R⁵ is hydrogen, halogen, or C₁₋₄ alkyl, and the C₁₋₄ alkyl is optionally substituted with one or more substituents selected from halogen, C₁₋₄ alkyl, -CH₂-O-CH₃, or -CH₂-O-C(=O)-CH₃;
preferably, R⁵ is hydrogen, halogen, or methyl.

4. The compound or the pharmaceutically acceptable form thereof according to any one of claims 1 to 3, wherein
L¹ is -NR⁶C(=O)-, -NR⁶S(=O)₂-, -C(=O)NR⁶-, or -S(=O)₂NR⁶-, and each R⁶ is independently hydrogen, C₁₋₄ alkyl, or C₃₋₆ cycloalkyl;
preferably, L¹ is -NR⁶C(=O)-, -NR⁶S(=O)₂-, -C(=O)NR⁶-, or -S(=O)₂NR⁶-, and each R⁶ is independently hydrogen or C₁₋₄ alkyl;
more preferably, L¹ is -NHC(=O)-, -N(CH₃)-C(=O)-, -NHS(=O)₂-, -C(=O)NH-, or - S(=O)₂NH-.

5. The compound or the pharmaceutically acceptable form thereof according to any one of claims 1 to 4, wherein
ring A is a 5-10 membered heteroaryl, ring A is optionally substituted with one or more R⁷, and each R⁷ is independently halogen, C₁₋₄ alkyl, or C₁₋₄ haloalkyl;
preferably, ring A is pyrrolyl, pyrazolyl, imidazolyl, thiazolyl, or oxazolyl, ring A is optionally substituted with one or more R⁷, and each R⁷ is independently halogen, methyl, ethyl, or trifluoromethyl;
more preferably, ring A is pyrrolyl or imidazolyl, ring A is optionally substituted with one or more R⁷, and each R⁷ is independently halogen or methyl;
particularly preferably, ring A is 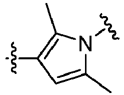 or

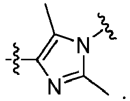

6. The compound or the pharmaceutically acceptable form thereof according to any one of claims 1 to 5, wherein
L² is a single bond, or CR⁸R⁹; R⁸ and R⁹ are each independently hydrogen, halogen, or C₁₋₄ alkyl;
preferably, L² is a single bond, -CH₂- or -CH(CH₃)-.

7. The compound or the pharmaceutically acceptable form thereof according to any one of claims 1 to 6, wherein
ring B is C₆₋₁₀ aryl or a 5-12 membered heteroaryl, ring B is optionally substituted with one or more R¹⁰, and
each R¹⁰ is independently halogen, cyano, amino, nitro, hydroxyl, C₁₋₆ alkyl, C₁₋₆ haloalkyl, C₁₋₆ alkoxy, C₁₋₆ haloalkoxy, C₃₋₆ cycloalkyl, or -C(=O)-NHz;
preferably, ring B is C₆₋₁₀ aryl or a 5-10 membered heteroaryl, ring B is optionally substituted with one or more R¹⁰, and each R¹⁰ is independently halogen, cyano, amino, nitro, hydroxyl, C₁₋₄ alkyl, C₁₋₄ alkoxy, or -C(=O)-NH₂;
more preferably, ring B is phenyl, pyrazinyl, pyridyl, pyrimidinyl, furanyl, oxazolyl, thienyl, thiazolyl, pyrazolyl, or imidazolyl, ring B is optionally substituted with one or more R¹⁰, and each R¹⁰ is independently halogen, cyano, amino, nitro, hydroxyl, methyl, or -C(=O)-NH₂;
particularly preferably, ring B is

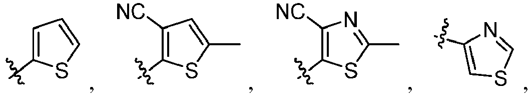

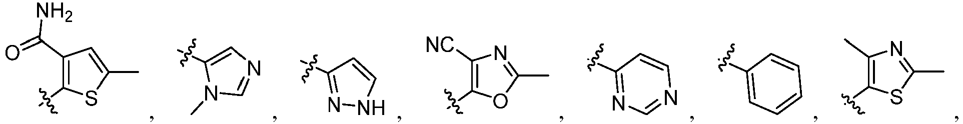

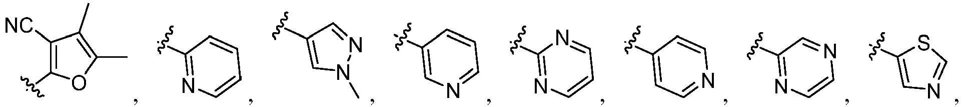

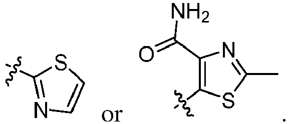

8. The compound or the pharmaceutically acceptable form thereof according to any one of claims 1 to 7, which is a compound having the structure of any one of formula 1-1, formula I-2, formula I-3, formula I-4, formula I-5, formula I-6, formula I-19 or formula I-20 or a pharmaceutically acceptable form thereof: 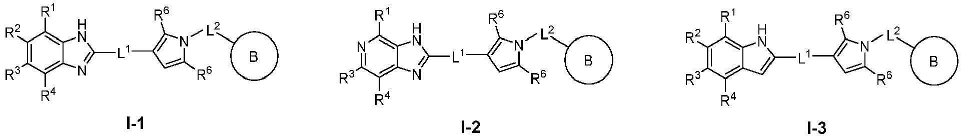 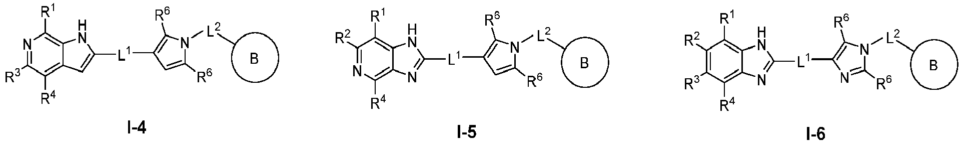 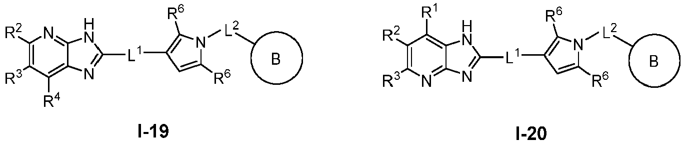 wherein R¹, R², R³, R⁴, R⁶, L¹, L² and ring B are as defined in any one of claims 1 to 7.

9. The compound or the pharmaceutically acceptable form thereof according to any one of claims 1 to 8, which is a compound having the structure of any one of formula I-7, formula I-8, formula I-9, formula I-10, formula I-11, formula I-12, formula I-13, formula I-14, formula I-15, formula I-16, formula I-17, formula I-18, formula I-21 or formula I-22 or a pharmaceutically acceptable form thereof: 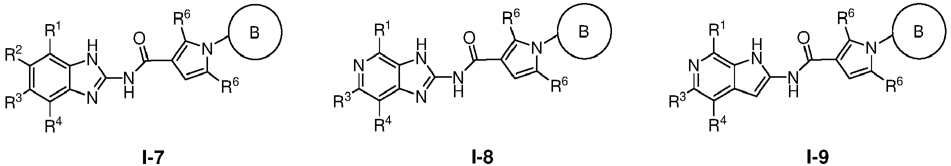 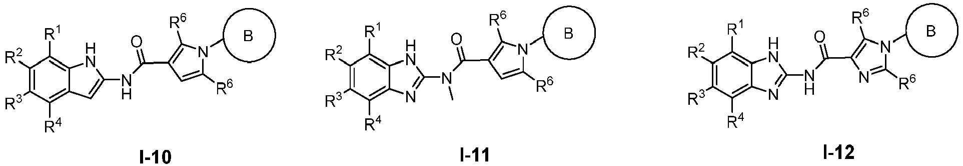 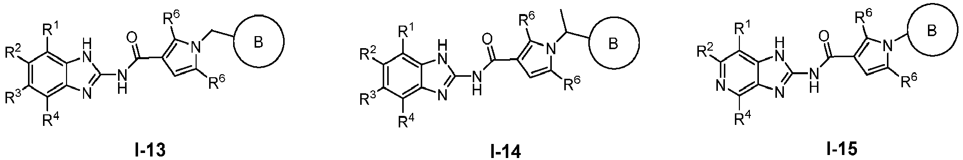 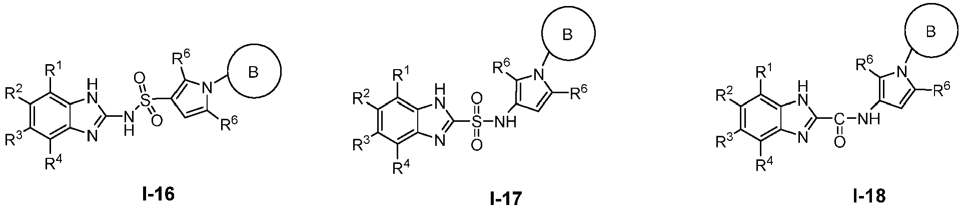 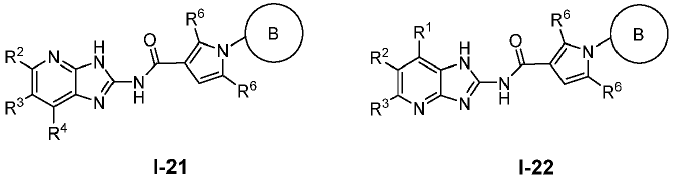 wherein R¹, R², R³, R⁴, R⁶, and ring B are as defined in any one of claims 1 to 8.

10. A compound or a pharmaceutically acceptable form thereof, the compound being selected from the following compounds: 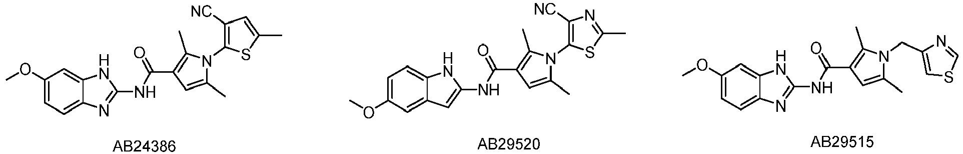 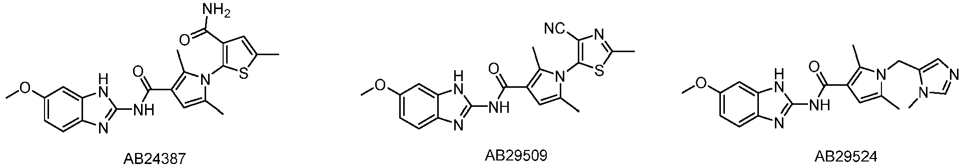 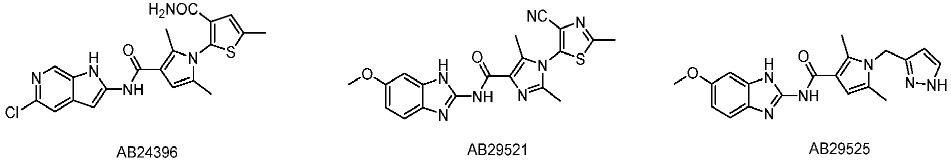 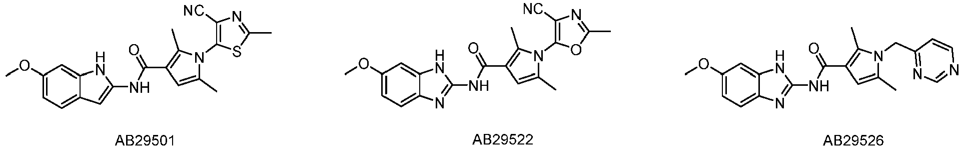 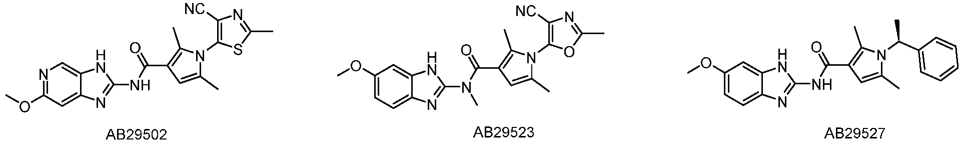 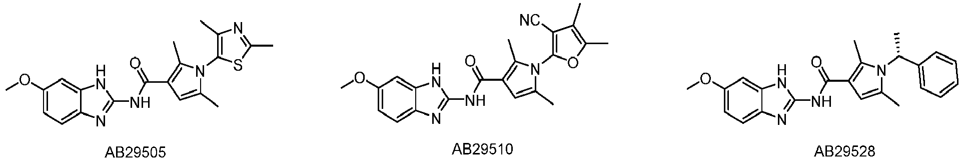 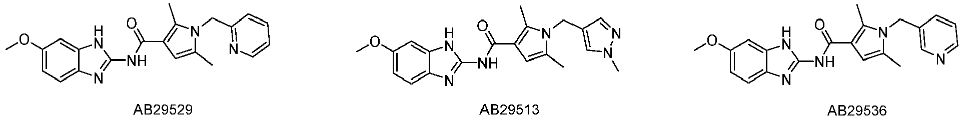 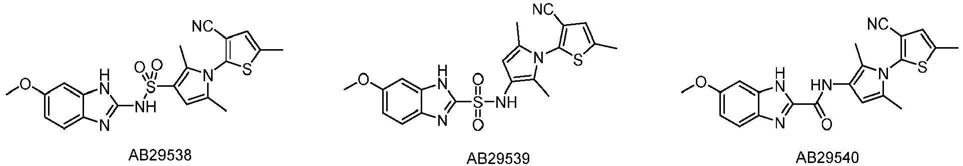 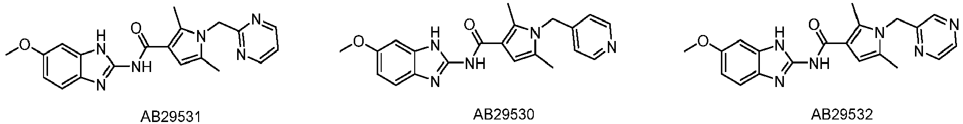 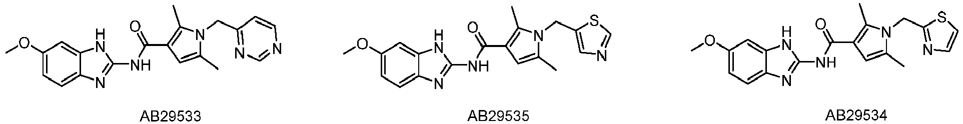 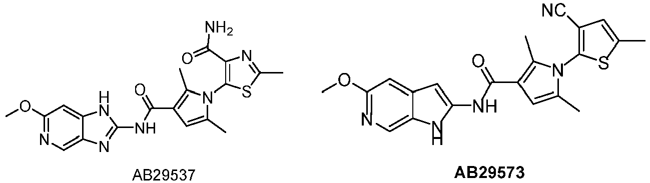 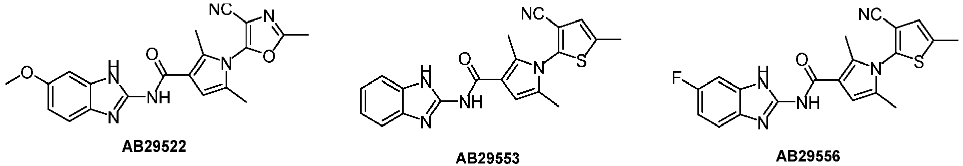 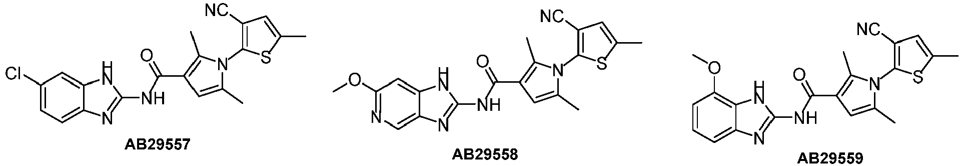 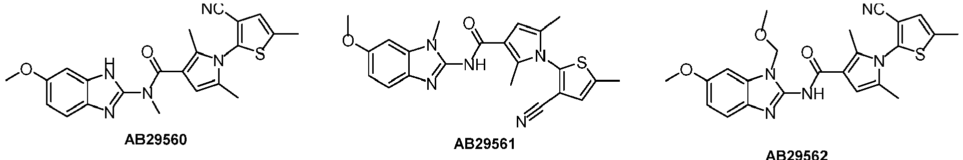 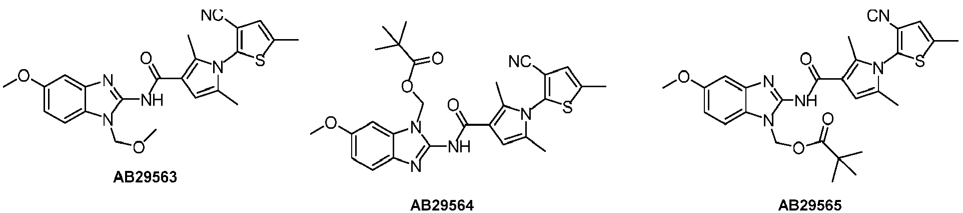 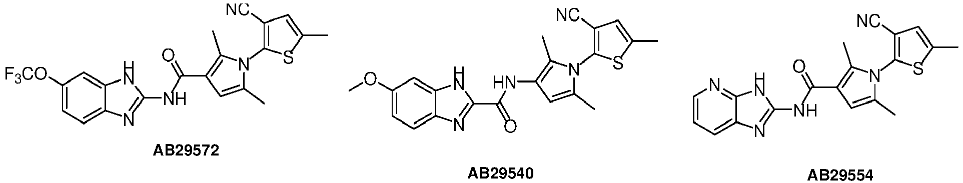 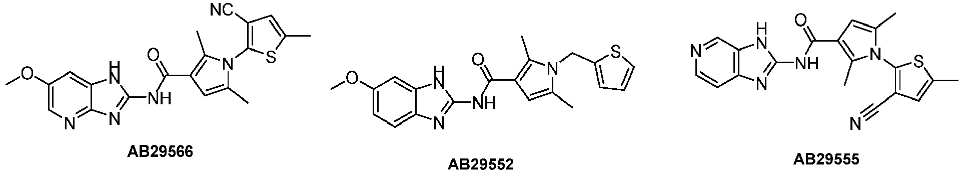 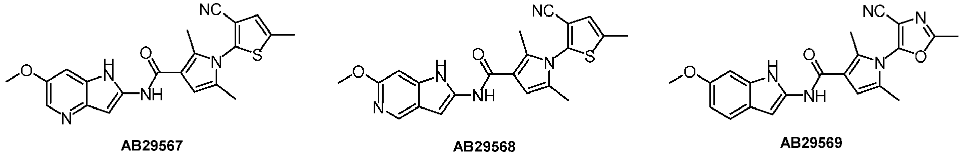 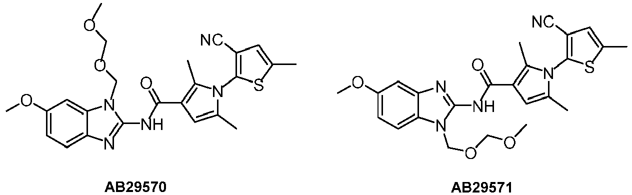 the pharmaceutically acceptable form is selected from a pharmaceutically acceptable salt, an ester, a stereoisomer, a tautomer, a solvate, a N-oxide, an isotopically labeled form, a metabolite and a prodrug.

11. A pharmaceutical composition, comprising the compound or the pharmaceutically acceptable form thereof according to any one of claims 1 to 10, and one or more pharmaceutically acceptable carriers.

12. Use of the compound or the pharmaceutically acceptable form thereof according to any one of claims 1 to 10 or the pharmaceutical composition according to claim 11 in preparation of a drug for preventing and/or treating a disease or a disorder at least partially mediated by DHX33.

13. The use according to claim 12, wherein the disease is selected from cancer, viral infection or inflammation that are mediated by DHX33.
